# EUROPEAN PATENT APPLICATION

(11) **EP 4 659 686 A1**
(43) Date of publication of application: **10.12.2025**
(21) Application number: 24749585.6
(22) Date of filing: 24.01.2024
(51) Int. Cl.: A61B 17/072, A61B 17/3209

(54) **SURGICAL INSTRUMENT**

(30) Priority: 02.02.2023 CN 202310101754; 02.02.2023 CN 202310071455; 02.02.2023 CN 202310073005
(71) Applicant: Fulbright Medical Inc., Wuxi, Jiangsu 214437 (CN)
(72) Inventor: SUN, Baofeng, Wuxi, Jiangsu 214437 (CN); WANG, Jisheng, Wuxi, Jiangsu 214437 (CN); ZHANG, Jianliang, Wuxi, Jiangsu 214437 (CN)
(74) Representative: Maiwald GmbH
(86) International application number: PCT/CN2024/073817
(87) International publication number: WO 2024/160104

(57) **Abstract**

Provided is a surgical instrument, comprising: an operating assembly, and a jaw assembly and a cutting knife assembly, which are connected to the operating assembly. The operating assembly comprises : a handle; a closing mechanism, which is connected to the jaw assembly; and a limiting member, which limits a fitting member when in a locked position, so as to prevent the fitting member from fitting with a cutting-knife driving member, wherein in response to first actuation of the handle, the closing mechanism drives the jaw assembly to switch from an open position to a closed position, keeps the jaw assembly in the closed position, and drives the limiting member to switch from the locked position to an unlocked position; and when in the unlocked position, the limiting member releases the limitation on the fitting member, so that the fitting member can fit with the cutting-knife driving member. The first actuation of the handle enables the jaw assembly to be closed for squeezing, and follow-up actuation of the handle enables a cutting knife to be fed for firing; thus, the surgical instrument has a simple overall structure, and is convenient to operate. In addition, the fitting member is always connected to the handle, and can stably drive the cutting-knife driving member, so that firing is more stable.

## Description

### CROSS-REFERENCE TO RELATED APPLICATION

For all purposes, the present patent application claims the priority to Chinese Patent Application No.202310071455.1 filed on February 2, 2023, Chinese Patent Application No.202310073005.6 filed on February 2, 2023, and Chinese Patent Application No.202310101754.5 filed on February 2, 2023, and the contents of the above-mentioned Chinese patent applications are incorporated herein by reference in their entireties.

### TECHNICAL FIELD

Embodiments of the present disclosure relate to a surgical instrument.

### BACKGROUND

Surgical cutting staplers are medical devices commonly used as alternatives to manual suturing. Their primary working mechanism involves the use of a cutting knife to cut and separate tissues and the use of titanium staples to perform stapling, resembling the function of a book stapler. These staplers are categorized into multiple types based on their application to different body regions. Specifically, the operational principle of surgical cutting staplers involves inserting a precisely positioned trocar cannula into the patient's body at the surgical site, subsequently creating longitudinal incisions in the tissue and applying staples on the opposite side of the incision, so as to achieve separation and stapling on tissue.

The surgical instrument includes a jaw assembly, a cutting knife assembly and an operating assembly, and the operating assembly is connected to the jaw assembly and the cutting knife assembly. During surgery, the operating assembly is manipulated by medical personnel to firstly close the jaw assembly to squeeze the patient's tissue, and then the cutting knife assembly can advance to cut the tissue, and simultaneously staples are fired from the staple cartridge for suturing.

### SUMMARY

In one aspect, an embodiment of the present disclosure provides a surgical instrument capable of controlling jaw closure and cutting knife advancement (firing) through a single handle, which has a simple structure, is easy to operate, and has a convenient and stable locking mechanism.

Embodiment of the present disclosure provide a surgical instrument, including: an operating assembly, and a jaw assembly and a cutting knife assembly respectively connected to the operating assembly, the operating assembly includes: a frame; a handle, rotatably connected to the frame, and configured to be operated to perform a first actuation and a subsequent actuation; a connecting rod assembly, including a first connecting rod and a second connecting rod, the first connecting rod being connected to the jaw assembly, one end of the second connecting rod is rotatably connected to the frame, and the other end of the second connecting rod is rotatably connected to the first connecting rod; in response to the first actuation of the handle, the connecting rod assembly switches from a first position to a second position, so as to drive the jaw assembly to be closed; when the connecting rod assembly is at the first position, the first connecting rod and the second connecting rod form an angle with each other, and when the connecting rod assembly is at the second position, the first connecting rod and the second connecting rod are collinear or substantially collinear, so that the connecting rod assembly is self-locked at the second position; a cutting knife driving member, connected to the cutting knife assembly; a mating member, connected to the handle; and a limiting member; when the connecting rod assembly is at the first position, the limiting member restrains the mating member so that the mating member disengages from the cutting knife driving member; when the connecting rod assembly is at the second position, the limiting member releases a restraint on the mating member, so as to enable the mating member to engage with the cutting knife driving member, and in response to the subsequent actuation of the handle, the mating member drives the cutting knife driving member to move, so as to drive the cutting knife assembly.

For example, the handle is provided with a supporting portion; the handle supports the first connecting rod or the second connecting rod through the supporting portion to drive the connecting rod assembly to move, so that the connecting rod assembly is switched from the first position to the second position; when the connecting rod assembly is at the second position, the supporting portion detaches from the connecting rod assembly when the handle performs the subsequent actuation.

For example, the limiting member is movably arranged on the frame, and the limiting member has a locked position and an unlocked position; when being at the locked position, the limiting member restrains the mating member to prevent engagement between the mating member and the cutting knife driving member; during switching from the first position to the second position, the connecting rod assembly drives the limiting member to move from the locked position to the unlocked position, and when the connecting rod assembly is at the second position, the limiting member is at the unlocked position; when being at the unlocked position, the limiting member releases the restraint on the mating member to enable the mating member to engage with the cutting knife driving member, and in response to the subsequent actuation of the handle, the mating member drives the cutting knife driving member to move, so as to drive the cutting knife assembly.

For example, when being at the locked position, the limiting member presses against the mating member to restrain the mating member; and when being at the unlocked position, the limiting member detaches from the mating member to release the restraint on the mating member, so as to enable the mating member to engage with the cutting knife driving member.

For example, the limiting member includes a body portion, a pressing portion and a protruding portion, the body portion is rotatably arranged on the frame, the pressing portion is connected to the body portion, and when the limiting member is at the locked position, the mating member is pressed by the pressing portion; the protruding portion is arranged on the body portion, and the connecting rod assembly drives the protruding portion, so as to drive the limiting member to switch from the locked position to the unlocked position.

For example, the limiting member is arranged on the first connecting rod or the second connecting rod, and in a process that the connecting rod assembly switches from the first position to the second position, the limiting member moves with the connecting rod assembly to switch from the locked position to the unlocked position, so as to release the restraint on the mating member.

For example, the limiting member includes a body portion and a blocking portion, the body portion is connected to the first connecting rod or the second connecting rod, the blocking portion is connected to the body portion and protrudes toward the mating member, and the blocking portion has an abutting surface; when the limiting member is at the locked position, the abutting surface abuts against the mating member to restrain the mating member; and in a process that the limiting member switches from the locked position to the unlocked position, the abutting surface and the mating member move relatively until the mating member detaches from the abutting surface, so as to release the restraint on the mating member.

For example, the limiting member includes a stopper, and when the limiting member is at the locked position, the stopper is located between the mating member and the cutting knife driving member to restrain the mating member, so as to separate the cutting knife driving member from the mating member; when the limiting member is at the unlocked position, the stopper detaches from a position between the mating member and the cutting knife driving member to release the restraint on the mating member, so as to enable the mating member to engage with the cutting knife driving member.

For example, when the limiting member is at the locked position, the stopper abuts against at least the mating member among the mating member and the cutting knife driving member.

For example, the limiting member further includes a movement portion movably arranged on the frame, the movement portion is connected to the stopper, and in a process that the connecting rod assembly drives the jaw assembly to switch from the open position to the closed position, the connecting rod assembly drives the movement portion to move, and the movement portion drives the stopper to move to detach from the position between the mating member and the cutting knife driving member.

For example, the movement portion is slidably arranged on the frame, the movement portion includes a sliding portion and a guide portion, the sliding portion is slidably connected to the frame, and the guide portion is connected to the sliding portion; in the process that the connecting rod assembly drives the jaw assembly to switch from the open position to the closed position, the connecting rod assembly abuts against and pushes the guide portion, so as to drive the movement portion to move.

For example, the mating member includes an extension protrusion, and when the connecting rod assembly is at the first position, the limiting member acts on the extension protrusion to restrain the mating member; when the connecting rod assembly is at the second position, the limiting member detaches from the extension protrusion to release the restraint on the mating member.

For example, the mating member is movably connected to the handle, an elastic member is provided between the handle and the mating member, and the mating member moves toward the cutting knife driving member under an action of an elastic force provided by the elastic member.

For example, the cutting knife driving member is a toothed member, the mating member includes a pawl, the pawl is rotatably connected to the handle, the elastic member is a torsion spring, and the pawl moves toward the cutting knife driving member under an action of an elastic force provided by the torsion spring.

In another aspect, an embodiment of the present disclosure provides a surgical instrument capable of controlling jaw closure and cutting knife advancement (firing) through a single handle, which has a simple structure and is easy to operate; and the handle will not get stuck and has high stability.

Embodiment of the present disclosure provide a surgical instrument, including: an operating assembly, and a jaw assembly and a cutting knife assembly respectively connected to the operating assembly, the operating assembly includes: a handle, operable to perform a first actuation and a subsequent actuation; a closing mechanism, connected to the jaw assembly; a cutting knife driving member, connected to the cutting knife assembly; a mating member, connected to the handle; and a limiting member, having a locked position and an unlocked position; when being at the locked position, the limiting member restrains the mating member to prevent engagement between the mating member and the cutting knife driving member; in response to the first actuation of the handle, the closing mechanism drives the jaw assembly to switch from an open position to a closed position and holds the jaw assembly at the closed position, and drives the limiting member to switch from the locked position to the unlocked position; when the jaw assembly is at the closed position, the limiting member is at the unlocked position; when the limiting member is at the unlocked position, the limiting member releases a restraint on the mating member to enable the mating member to engage with the cutting knife driving member, and in response to the subsequent actuation of the handle, the mating member drives the cutting knife driving member to move, so as to drive the cutting knife assembly.

For example, when being at the locked position, the limiting member presses against the mating member to restrain the mating member; and when being at the unlocked position, the limiting member detaches from the mating member to release the restraint on the mating member, so as to enable the mating member to engage with the cutting knife driving member.

For example, the operating assembly further includes a frame, the limiting member is movably arranged on the frame, and when the limiting member switches from the locked position to the unlocked position, the limiting member moves to detach from the mating member.

For example, the limiting member includes a body portion, a pressing portion and a protruding portion, the body portion is rotatably arranged on the frame, the pressing portion is connected to the body portion, and when the limiting member is at the locked position, the pressing portion presses against the mating member; the protruding portion is arranged on the body portion, and the closing mechanism abuts against the protruding portion to drive the protruding portion, so as to further drive the limiting member to switch from the locked position to the unlocked position.

For example, the limiting member includes a stopper, and when the limiting member is at the locked position, the stopper is located between the mating member and the cutting knife driving member, so as to restrain the mating member and separate the cutting knife driving member from the mating member; when the limiting member is at the unlocked position, the stopper detaches from a position between the mating member and the cutting knife driving member to release the restraint on the mating member, so as to enable the mating member to engage with the cutting knife driving member.

For example, when the limiting member is at the locked position, the stopper abuts against at least the mating member among the mating member and the cutting knife driving member.

For example, the operating assembly further includes a frame, the limiting member further includes a movement portion movably arranged on the frame, the movement portion is connected to the stopper, and in a process that the closing mechanism drives the jaw assembly to switch from the open position to the closed position, the closing mechanism drives the movement portion to move, and the movement portion drives the stopper to move to detach from the position between the mating member and the cutting knife driving member.

For example, the movement portion is slidably arranged on the frame, the movement portion includes a sliding portion and a guide portion, the sliding portion is slidably connected to the frame, and the guide portion is connected to the sliding portion; in the process that the closing mechanism drives the jaw assembly to switch from the open position to the closed position, the closing mechanism abuts against and pushes the guide portion, so as to drive the movement portion to move.

For example, the mating member is movably connected to the handle, an elastic member is provided between the handle and the mating member, and the mating member moves toward the cutting knife driving member under an action of an elastic force provided by the elastic member.

For example, the cutting knife driving member is a toothed member, the mating member includes a pawl, the pawl is rotatably connected to the handle, the elastic member is a torsion spring, and the pawl moves toward the cutting knife driving member under an action of an elastic force provided by the torsion spring.

For example, the closing mechanism includes a shaft driving member and a shaft assembly, the shaft assembly is connected to the jaw assembly, the shaft driving member is connected to the shaft assembly, when the handle performs the first actuation, the handle is operably engaged with the shaft driving member to drive the shaft driving member to switch from a first position to a second position, so as to further drive the shaft assembly to move and to cause the jaw assembly to switch from the open position to the closed position; and during switching from the first position to the second position, the shaft driving member drives the limiting member to switch from the locked position to the unlocked position.

For example, the closing mechanism further includes a locking member, and when the shaft driving member is at the second position, the locking member locks the shaft driving member to hold the jaw assembly at the closed position.

For example, the locking member includes a pin, the pin abuts against the shaft driving member when the shaft driving member is at the second position, so as to block a movement of the shaft driving member towards the first position and to lock the shaft driving member.

For example, the shaft driving member includes a connecting rod assembly, the connecting rod assembly includes a first connecting rod and a second connecting rod, the first connecting rod is rotatably connected to the shaft assembly, one end of the second connecting rod is rotatably connected to the first connecting rod, and the other end of the second connecting rod is rotatably connected to the frame; the handle is in contact with the first connecting rod or the second connecting rod, such that the handle, during the first actuation, drives the connecting rod assembly to switch from the first position to the second position; when the connecting rod assembly is at the second position, the connecting rod assembly is at or substantially at a dead center, so that the connecting rod assembly is locked at the second position.

For example, the handle is provided with a supporting portion; the handle supports the first connecting rod or the second connecting rod through the supporting portion to drive the connecting rod assembly to move, so that the connecting rod assembly is switched from the first position to the second position; when the connecting rod assembly is at the second position, the supporting portion detaches from the connecting rod assembly when the handle performs the subsequent actuation.

In yet another aspect, an embodiment of the present disclosure provides a surgical instrument that increases the stability of jaw assembly closure and the stability of tissue squeeze.

Embodiment of the present disclosure provide a surgical instrument, including: an operating assembly, and a jaw assembly connected to the operating assembly, the operating assembly includes: a frame; a handle, rotatably connected to the frame; a connecting rod assembly, including a left connecting rod and a right connecting rod, the left connecting rod and the right connecting rod are respectively located on both sides of the frame, the left connecting rod is connected to the right connecting rod, and the handle is operatively engaged with the left connecting rod and/or the right connecting rod to drive the connecting rod assembly to move; in response to movement of the connecting rod assembly, the jaw assembly moves from an open position to a closed position.

For example, the left connecting rod includes a left first connecting rod and a left second connecting rod, the left first connecting rod is connected to the jaw assembly, one end of the left second connecting rod is rotatably connected to the frame, and the other end of the left second connecting rod is rotatably connected to the left first connecting rod; the right connecting rod includes a right first connecting rod and a right second connecting rod, the right first connecting rod is connected to the jaw assembly, one end of the right second connecting rod is rotatably connected to the frame, and the other end of the right second connecting rod is rotatably connected to the right first connecting rod; and the left second connecting rod is connected to the right second connecting rod.

For example, the handle drives the connecting rod assembly to move from the first position to the second position, when the connecting rod assembly is at the first position, the left first connecting rod and the left second connecting rod form a first angle with each other, and the right first connecting rod and the right second connecting rod form the first angle with each other; when the connecting rod assembly is at the second position, the left first connecting rod is collinear or substantially collinear with the left second connecting rod, and the right first connecting rod is collinear or substantially collinear with the right second connecting rod.

For example, the handle is operable to perform a first actuation and a subsequent actuation; the handle, during the first actuation, drives the connecting rod assembly to move from the first position to the second position, the operating assembly further includes a cutting knife driving member, connected to the cutting knife assembly; a mating member, connected to the handle; and a limiting member; when the connecting rod assembly is at the first position, the limiting member restrains the mating member so that the mating member disengages from the cutting knife driving member; when the connecting rod assembly is at the second position, the limiting member releases a restraint on the mating member, so as to enable the mating member to engage with the cutting knife driving member, and in response to the subsequent actuation of the handle, the mating member drives the cutting knife driving member to move, so as to drive the cutting knife assembly.

For example, the mating member is movably connected to the handle, an elastic member is provided between the handle and the mating member, and the mating member moves toward the cutting knife driving member under an action of an elastic force provided by the elastic member.

For example, the cutting knife driving member is a toothed member, the mating member includes a pawl, the pawl is rotatably connected to the handle, the elastic member is a torsion spring, and the pawl moves toward the cutting knife driving member under an action of an elastic force provided by the torsion spring.

For example, the limiting member is movably arranged on the frame, when being at the locked position, the limiting member restrains the mating member to prevent engagement between the mating member and the cutting knife driving member; during switching from the first position to the second position, the connecting rod assembly drives the limiting member to move from the locked position to the unlocked position, and when the connecting rod assembly is at the second position, the limiting member is at the unlocked position; when being at the unlocked position, the limiting member releases the restraint on the mating member to enable the mating member to engage with the cutting knife driving member, and in response to the subsequent actuation of the handle, the mating member drives the cutting knife driving member to move, so as to drive the cutting knife assembly.

For example, the limiting member includes a body portion, a pressing portion and a protruding portion, the body portion is rotatably arranged on the frame, the pressing portion is connected to the body portion, and when the limiting member is at the locked position, the pressing portion presses against the mating member; the protruding portion is arranged on the body portion, and the closing mechanism abuts against the protruding portion to drive the protruding portion, so as to further drive the limiting member to switch from the locked position to the unlocked position.

For example, the limiting member includes a stopper, and when the limiting member is at the locked position, the stopper is located between the mating member and the cutting knife driving member to restrain the mating member, so as to separate the cutting knife driving member from the mating member; when the limiting member is at the unlocked position, the stopper detaches from a position between the mating member and the cutting knife driving member to release the restraint on the mating member, so as to enable the mating member to engage with the cutting knife driving member.

For example, when the limiting member is at the locked position, the stopper abuts against at least the mating member among the mating member and the cutting knife driving member.

For example, the limiting member further includes a movement portion movably arranged on the frame, the movement portion is connected to the stopper, and in a process that the closing mechanism drives the jaw assembly to switch from the open position to the closed position, the closing mechanism drives the movement portion to move, and the movement portion drives the stopper to move to detach from the position between the mating member and the cutting knife driving member.

For example, the movement portion is slidably arranged on the frame, the movement portion includes a sliding portion and a guide portion, the sliding portion is slidably connected to the frame, and the guide portion is connected to the sliding portion; in the process that the closing mechanism drives the jaw assembly to switch from the open position to the closed position, the closing mechanism abuts against and pushes the guide portion, so as to drive the movement portion to move.

For example, the limiting member is arranged on the left connecting rod or the right connecting rod, and in a process that the connecting rod assembly switches from the first position to the second position, the limiting member moves with the connecting rod assembly to switch from the locked position to the unlocked position, so as to release the restraint on the mating member.

For example, the connecting rod assembly further includes a connecting portion, one end of the connecting portion is rotatably connected to the left second connecting rod, and the other end of the connecting portion is rotatably connected to the right second connecting rod.

For example, the connecting portion includes a clamp plate, a left hinge portion and a right hinge portion, the clamp plate is connected to the frame, the left hinge portion and the right hinge portion are respectively arranged at both sides of the clamp plate, the left hinge portion is rotatably connected to the left second connecting rod, and the right hinge portion is rotatably connected to the right second connecting rod.

For example, the left first connecting rod includes a left first hinge hole and a left middle hinge hole, the left first connecting rod is rotatably connected to the jaw assembly through the left first hinge hole, and the left first connecting rod is rotatably connected to the left second connecting rod through the left middle hinge hole; the right first connecting rod includes a right first hinge hole and a right middle hinge hole, the right first connecting rod is rotatably connected to the jaw assembly through the right first hinge hole, and the right first connecting rod is rotatably connected to the right second connecting rod through the right middle hinge hole; the left first hinge hole is coaxially arranged with the right first hinge hole, and the left middle hinge hole is coaxially arranged with the right middle hinge hole.

For example, the operating assembly further includes an outer cannula, the outer cannula is connected to the jaw assembly, and both the left first connecting rod and the right first connecting rod are rotatably connected to the outer cannula.

For example, the handle includes a handle body and a supporting portion connected to the handle body, the supporting portion supports the left connecting rod and/or the right connecting rod, so that the handle is operably engaged with the left connecting rod and/or the right connecting rod.

### BRIEF DESCRIPTION OF DRAWINGS

In order to clearly illustrate the technical solution of the embodiments of the present disclosure, the drawings of the embodiments will be briefly described in the following. Clearly, the described drawings are only related to some embodiments of the present disclosure and thus are not construed as any limitation to the present disclosure.
Fig. 1 is a schematic structural diagram of a surgical instrument according to an embodiment of the present disclosure;
Fig. 2 is a schematic structural diagram of a surgical instrument before first actuation according to an embodiment of the present disclosure;
Fig. 3 is a schematic structural diagram of a surgical instrument after first actuation according to an embodiment of the present disclosure;
Fig. 4 is another schematic structural diagram of a surgical instrument when a protruding portion is in contact with a connecting rod assembly according to an embodiment of the present disclosure;
Fig. 5 is another schematic structural diagram of a protruding portion of a surgical instrument when a connecting rod assembly is at a second position according to an embodiment of the present disclosure;
Fig. 6a is a schematic structural diagram of a surgical instrument when a limiting member is at a locked position according to an embodiment of the present disclosure;
Fig. 6b is a schematic structural diagram of a surgical instrument when a limiting member is at a locked position according to an embodiment of the present disclosure;
Fig. 6c is a partial view of position A in Fig. 6b;
Fig. 7 is a schematic structural diagram of a surgical instrument when a limiting member is at an unlocked position according to an embodiment of the present disclosure;
Fig. 8 is a schematic structural diagram of a mating member of a surgical instrument according to an embodiment of the present disclosure;
Fig. 9 is a schematic structural diagram of a surgical instrument when a mating member engages with a cutting knife driving member according to an embodiment of the present disclosure;
Fig. 10 is a schematic structural diagram of a surgical instrument when a handle performs a subsequent actuation to drive a cutting knife assembly according to an embodiment of the present disclosure;
Fig. 11 is a schematic structural diagram of a cutting knife assembly and a cutting knife driving member of a surgical instrument according to an embodiment of the present disclosure;
Fig. 12 is a schematic structural diagram of a pawl and a handle of a surgical instrument according to an embodiment of the present disclosure;
Fig. 13 is a schematic structural diagram of a surgical instrument when a pawl does not mesh with a cutting knife driving member according to an embodiment of the present disclosure;
Fig. 14 is a schematic structural diagram of another limiting member of a surgical instrument according to an embodiment of the present disclosure;
Fig. 15 is a schematic structural diagram of a limiting member of a surgical instrument from another viewing angle according to an embodiment of the present disclosure;
Fig. 16 is a schematic structural diagram of a surgical instrument when a limiting member is at a locked position according to an embodiment of the present disclosure;
Fig. 17 is a cross-sectional view of a surgical instrument when a limiting member is at a locked position according to an embodiment of the present disclosure;
Fig. 18 is a schematic structural diagram of a surgical instrument when a limiting member moves to an unlocked position according to an embodiment of the present disclosure;
Fig. 19 is a schematic structural diagram of a surgical instrument when a limiting member is at an unlocked position according to an embodiment of the present disclosure;
Fig. 20 is a cross-sectional view of a surgical instrument when a limiting member of the surgical instrument is at an unlocked position according to an embodiment of the present disclosure;
Fig. 21 is a schematic structural diagram of further another limiting member of a surgical instrument according to an embodiment of the present disclosure;
Fig. 22 is a schematic structural diagram of a surgical instrument when a limiting member is at a locked position according to an embodiment of the present disclosure;
Fig. 23 is a schematic structural diagram of a surgical instrument when a limiting member switches to an unlocked position according to an embodiment of the present disclosure;
Fig. 24 is a schematic structural diagram of a surgical instrument when a limiting member is at an unlocked position according to an embodiment of the present disclosure;
Fig. 25 is a schematic structural diagram of a surgical instrument when a locking member does not lock a connecting rod assembly according to an embodiment of the present disclosure;
Fig. 26 is a schematic structural diagram of a surgical instrument when a locking member locks a connecting rod assembly according to an embodiment of the present disclosure;
Fig. 27 is a schematic structural diagram of a connecting rod assembly and a locking member of a surgical instrument according to an embodiment of the present disclosure;
Fig. 28 is a schematic structural diagram of a surgical instrument when a connecting rod assembly is in contact with a locking member according to an embodiment of the present disclosure;
Fig. 29 is a schematic structural diagram of a surgical instrument after a connecting rod assembly passes over a locking member according to an embodiment of the present disclosure;
Fig. 30 is a schematic structural diagram of a handle of a surgical instrument according to an embodiment of the present disclosure;
Fig. 31 is a schematic structural diagram of a surgical instrument according to an embodiment of the present disclosure;
Fig. 32 is a schematic structural diagram of a connecting rod assembly of a surgical instrument according to an embodiment of the present disclosure;
Fig. 33 is a schematic structural diagram of a left connecting rod and a right connecting rod of a surgical instrument according to an embodiment of the present disclosure;
Fig. 34 is a top view of a connecting rod assembly of a surgical instrument according to an embodiment of the present disclosure;
Fig. 35 is a schematic structural diagram of a connecting member and a connecting rod assembly of a surgical instrument according to an embodiment of the present disclosure;
Fig. 36 is a partial view of position A in Fig. 35;
Fig. 37 is a cross-sectional view taken along line A-A' in Fig. 32;
Fig. 38 is a schematic structural diagram of a surgical instrument before first actuation according to an embodiment of the present disclosure;
Fig. 39 is a schematic structural diagram of a surgical instrument after first actuation according to an embodiment of the present disclosure.

### DETAILED DESCRIPTION

In order to make objectives, technical details, and advantages of the embodiments of the present disclosure more clear, the technical solutions of the embodiments will be described in a clearly and fully understandable way in connection with the drawings related to the embodiments of the present disclosure. It is clear that the described embodiments are just a part but not all of the embodiments of the present disclosure. Based on the described embodiments herein, those skilled in the art can obtain other embodiment(s), without any inventive work, which should be within the scope of the present disclosure.

It is to be understood that the terms "proximal" and "distal" used herein are defined relative to the clinician manipulating the handle of the stapler. The term "proximal" refers to a portion close to the clinician, while the term "distal" refers to a portion away from the clinician. That is, the handle is proximal and the jaw assembly is distal. For example, a proximal end of a component represents an end relatively close to the handle, and a distal end of the component represents an end relatively close to the jaw assembly. The terms "above" and "below" are defined according to the relative positions of the staple abutting seat and the staple cartridge seat of the jaw assembly. Specifically, the staple abutting seat is positioned "above" and the staple cartridge seat is positioned "below". However, the stapler can be used in many orientations and positions, and thus these terms expressing relative positional relationships are not restrictive and absolute.

In the embodiments of the present disclosure, unless otherwise clearly stated and defined, the terms "connected," "coupled," and the like are to be understood in a broad sense; for example, it can mean fixed connection, detachable connection, movable connection, or integrated configuration; it can be direct connection or indirect connection through an intermediate medium, it can be internal communication between two elements or interaction relationship of two elements, such as abutment. For those skilled in the art, the specific meanings of these terms in the embodiments of the present disclosure can be understood according to actual circumstances. It should be noted that when there is a qualifier before "connected" or "coupled", it has the meaning defined by the corresponding qualifier, excluding only the situations that obviously need to be excluded and not excluding other possible situations. For example, "detachably connected" refers to a detachable connection, which does not include integrated configuration, but movable connection and the like are not excluded.

In common technology, the operating assembly is generally provided with two handles to respectively control jaw closure and cutting knife advancement, resulting in a complex structure and cumbersome operation.

In common technology, there exists a stapler with a single handle that can control jaw closure and cutting knife advancement, which includes a handle, a cam, a connecting rod, a rack gear and a ratchet assembly. The handle is connected to the cam. Before the handle performs a first actuation, the ratchet assembly disengages from the handle, and the ratchet assembly disengages from the rack gear. When the handle performs the first actuation, the cam rotates and drives the connecting rod to move to close the jaws. After rotation, the cam is locked by a spring mechanism. When the cam rotates, the cam creates space for the ratchet assembly to move, the moved ratchet assembly meshes with the handle and the rack gear simultaneously, allowing the handle to drive the jaw rack gear during a subsequent actuation, and thus driving the cutting knife. The structure in which the cam is locked through the spring assembly is complex, and the locking stability is poor, which can easily lead to locking errors and cause the jaws to reopen.

An embodiment of the present disclosure provides a surgical instrument, which can be a stapler, and includes an operating assembly, and a jaw assembly and a cutting knife assembly respectively connected to the operating assembly; the operating assembly includes a closing mechanism, a handle, a cutting knife driving member, a mating member and a limiting member; the closing mechanism is connected to the jaw assembly, the cutting knife driving member is connected to the cutting knife assembly; and the mating member is connected to the handle and configured to engage with the cutting knife driving member. The handle is operable to perform a first actuation and a subsequent actuation, the first actuation of the handle can cause the closing mechanism to drive the jaw assembly to switch from an open position to a closed position, the subsequent actuation is performed after the first actuation, and during the subsequent actuation, the cutting knife assembly is driven to advance for firing.

The closing mechanism includes a connecting rod assembly. The connecting rod assembly includes a first connecting rod and a second connecting rod, the first connecting rod is connected to the jaw assembly, one end of the second connecting rod is rotatably connected to a frame, and the other end of the second connecting rod is rotatably connected to the first connecting rod; in response to the first actuation of the handle, the connecting rod assembly switches from a first position to a second position, so as to drive the jaw assembly to be closed; when the connecting rod assembly is at the first position, the first connecting rod and the second connecting rod form an angle with each other, and when the connecting rod assembly is at the second position, the first connecting rod and the second connecting rod are collinear or substantially collinear, so that the connecting rod assembly is self-locked at the second position.

Collinear means that the first connecting rod and the second connecting rod are located on a same straight line, and an included angle between the first connecting rod and the second connecting rod is 180 degrees. Substantially collinear means that the first connecting rod and the second connecting rod go beyond a dead center position, and the included angle between the first connecting rod and the second connecting rod is greater than 0 degree and less than 5 degrees. When the connecting rod assembly is at the dead center (corresponding to the collinear case) or substantially at the dead center (corresponding to the substantially collinear case), the pressure angle between the first connecting rod and the second connecting rod is substantially equal to 90 degrees, and when the first connecting rod or the second connecting rod is subjected to external force, the torque exerted on the other connecting rod is zero, so that the connecting rod assembly cannot move, and the connecting rod assembly is self-locked at the second position. Therefore, the jaw assembly is locked at the closed position.

The limiting member has a locked position and an unlocked position. When being at the locked position, the limiting member restrains the mating member to prevent engagement between the mating member and the cutting knife driving member, and the restraint means that the limiting member exerts force on the mating member to prevent engagement between the mating member and the cutting knife driving member. When being at the unlocked position, the limiting member releases the restraint on the mating member; specifically, no force is exerted on the mating member, so that the mating member can engage with the cutting knife assembly.

Before the first actuation, the jaw assembly is at the open position, and the limiting member is at the locked position; in response to the first actuation of the handle, the closing mechanism drives the jaw assembly to switch from the open position to the closed position and holds the jaw assembly at the closed position, i.e., achieves closure of the jaw assembly and maintains the squeeze; at the same time, the closing mechanism drives the limiting member to switch from the locked position to the unlocked position, when the closing mechanism holds the jaw assembly at the closed position, the limiting member is at the unlocked position, and the mating member can engage with the cutting knife driving member.

It should be noted that during the first actuation of the handle, the limiting member is at the locked position, and the mating member does not engage with the cutting knife driving member; therefore, the first actuation of the handle can only drive the jaw assembly to be closed and not drive the cutting knife to advance. Aft the completion of the first actuation, the limiting member is at the unlocked position, the mating member can engage with the cutting knife driving member, and when the handle performs the subsequent actuation, the mating member and the cutting knife driving member can drive the cutting knife assembly to advance for firing.

When the handle performs the subsequent actuation, the mating member and the cutting knife driving member drive the cutting knife assembly to advance for firing. At the same time, the handle does not act on the closing mechanism, the jaw assembly remains at the closed position, and the limiting member remains at the unlocked position.

The surgical instrument provided by the embodiment of the present disclosure is only provided with one handle, the handle causes the jaw assembly to be closed and to squeeze during the first actuation, and causes the cutting knife to advance for firing during the subsequent actuation, the entire structure is simple, and the operation is convenient.

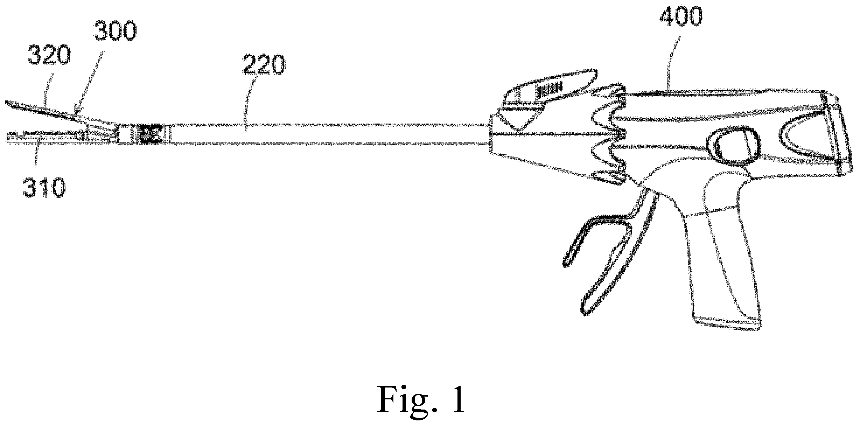

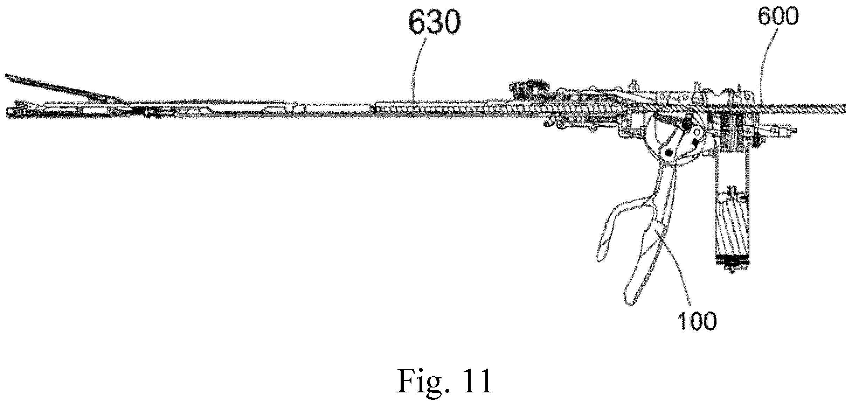

An embodiment of the present disclosure provides a surgical instrument, which can be a stapler. As shown in Figs. 1 and 11, the surgical instrument includes an operating assembly 400, and a jaw assembly 300 and a cutting knife assembly 630 respectively connected to the operating assembly 400, the jaw assembly 300 includes a staple cartridge seat 310 and a staple abutting seat 320, a staple cartridge assembly (not shown) is detachably mounted in the staple cartridge seat 310, the staple abutting seat 320 is rotatably connected to the staple cartridge seat 310, and the rotation of the staple abutting seat 320 causes the jaw assembly 300 to have an open position and a closed position; in response to the medical personnel's operation on the operating assembly 400, the jaw assembly 300 can switch between the open position and the closed position; when the jaw assembly 300 is at the open position, the tissue can enter into the jaw assembly 300, and when the jaw assembly 300 is at the closed position, the tissue can be squeezed. When the tissue is in the squeezed state, the cutting knife assembly 630 advances to cut the tissue in response to the medical personnel's operation on the operating assembly 400, and the staple cartridge assembly outputs staples to suture the cut tissue.

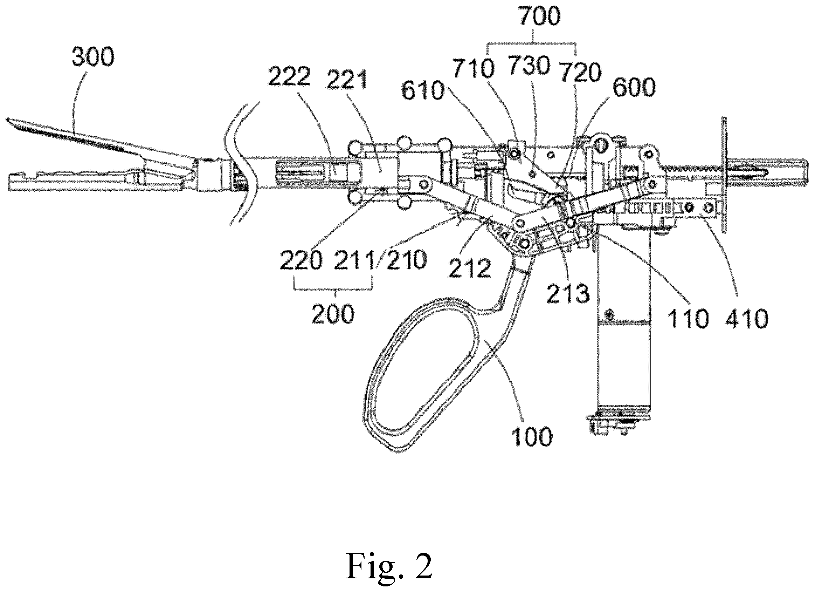

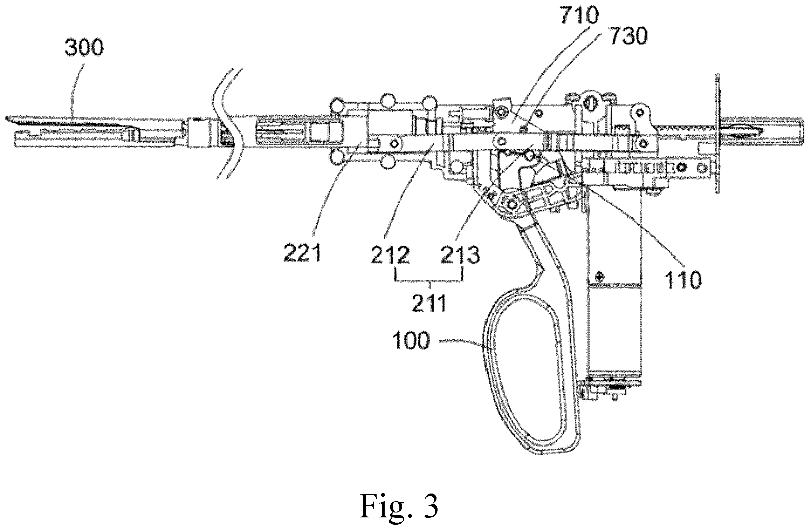

For example, as shown in Figs. 2 and 3, the operating assembly 400 includes a frame 410, a handle 100, a closing mechanism 200, a cutting knife driving member 600, a mating member 610, and a limiting member 700. For example, the handle 100 is rotatably arranged on the frame 410, an elastic assembly is arranged between the handle 100 and the frame 410, and the handle 100 has an initial position and a pressed position. The medical personnel can cause the handle 100 to move from the initial position to the pressed position by pressing the handle 100. When the handle 100 is released, the handle 100 can rebound from the pressed position to the initial position under the action of the elastic assembly. The process of the handle 100 moving from the initial position to the pressed position is called actuation. During surgery, the handle 100 is operated to perform multiple actuations, including a first actuation and a subsequent actuation, the subsequent actuation is an actuation other than the first actuation, the first actuation can drive the jaw assembly 300 to switch to the closed position to squeeze the tissue, and the subsequent actuation can drive the cutting knife assembly 630 to advance for firing.

For example, as shown in Fig. 2, the closing mechanism 200 includes a connecting rod assembly 211 and a shaft assembly 220, the shaft assembly 220 is connected to the jaw assembly 300, and the connecting rod assembly 211 is connected to the shaft assembly 220. When the handle 100 performs the first actuation, the handle 100 is operably engaged with the connecting rod assembly 211 to enable the handle 100 to drive the connecting rod assembly 211 to move, and when the connecting rod assembly 211 moves, it can drive the shaft assembly 220 to move, so as to cause the jaw assembly 300 to switch from the open position to the closed position. For example, the shaft assembly 220 includes an inner cannula 222 and an outer cannula 221, the outer cannula 221 is looped around the inner cannula 222, and the outer cannula 221 can move relative to the inner cannula 222; one end of the outer cannula 221 is connected to the connecting rod assembly 211, and the other end of the outer cannula 221 is connected to the jaw assembly 300. In response to the first actuation of the handle 100, the connecting rod assembly 211 switches from the first position to the second position to drive the outer cannula 221 to move, and the movement of the outer cannula 221 causes the jaw assembly 300 to switch from the open position to the closed position. When the handle 100 is switched to the pressed position, the connecting rod assembly 211 is locked at the second position, which in turn locks the position of the outer cannula 221, and the jaw assembly 300 remains at the closed position. When the handle 100 rebounds from the pressed position to the initial position, the connecting rod assembly 211 remains at the second position and does not move with the handle 100, and the outer cannula 221 also remains at the position that causes the jaw assembly 300 to be at the closed position.

As shown in Fig. 2, the connecting rod assembly 211 includes a first connecting rod 212 and a second connecting rod 213; the first connecting rod 212 is rotatably connected to the shaft assembly 220, specifically to the outer cannula 221; one end of the second connecting rod 213 is rotatably connected to the frame 410, and the other end of the second connecting rod 213 is rotatably connected to the first connecting rod 212, so as to form a dual-link mechanism. As shown in Fig. 2, the connecting rod assembly 211 is at the first position, and when the connecting rod assembly 211 is at the first position, the first connecting rod 212 and the second connecting rod 213 form an angle with each other, and both the first connecting rod 212 and the second connecting rod 213 are inclined toward the lower side (a side close to the gripping portion of the handle 100). When the connecting rod assembly 211 is at the first position and the handle 100 has not performed the first actuation, the handle 100 is in contact with the first connecting rod 212 or the second connecting rod 213 to enable the handle 100 to drive the connecting rod assembly 211 to move. For example, the distal end of the first connecting rod 212 is rotatably connected to the outer cannula 221, the proximal end of the first connecting rod 212 is rotatably connected to the second connecting rod 213, and the first connecting rod 212 is rotatably connected to the second connecting rod 213 through a hinge point. In the process that the connecting rod assembly 211 switches from the first position to the second position, the hinge point gradually moves to the upper side (a side away from the gripping portion of the handle 100), and because the proximal end of the second connecting rod 213 is connected to the frame 410, the hinge point located at the distal end of the second connecting rod 213 moves toward the distal end; at the same time, the rotation of the first connecting rod 212 causes the distal end of the first connecting rod 212 to move toward the distal end, and therefore, the connecting rod assembly 211 can drive the outer cannula 221 to move toward the distal end, so as to close the jaw assembly 300.

As shown in Fig. 3, the connecting rod assembly 211 is at the second position, and when the connecting rod assembly 211 is at the second position, the first connecting rod 212 and the second connecting rod 213 of the connecting rod assembly 211 are collinear or substantially collinear. Collinear means that the first connecting rod 212 and the second connecting rod 213 are located on a same straight line, and an included angle between the first connecting rod 212 and the second connecting rod 213 is 180 degrees. Substantially collinear means that the first connecting rod 212 and the second connecting rod 213 go beyond a dead center position, and the included angle between the first connecting rod 212 and the second connecting rod 213 is greater than 0 degree and less than 5 degrees. When the connecting rod assembly 211 is at the dead center, the pressure angle between the first connecting rod 212 and the second connecting rod 213 is substantially equal to 90 degrees, and when the first connecting rod 212 or the second connecting rod 213 is subjected to an external force, the torque exerted on the other connecting rod is zero, so that the connecting rod assembly 211 cannot move, and the connecting rod assembly 211 is self-locked at the second position. The self-lock of the connecting rod assembly 211 at the second position maintains the jaw assembly 300 at the closed position, that is, the connecting rod assembly can be self-locked after driving the jaw assembly to be closed, so that the jaw assembly is locked at the closed position, and the self-lock of the connecting rod assembly 211 does not require operation and does not require the setting of a locking structure, so that the entire structure of the surgical instrument is simple, the locking of the connecting rod assembly 211 is stable, and the locking failure is not easy to occur at the same time.

As shown in Figs. 2 and 3, in response to the first actuation of the handle 100, the connecting rod assembly 211 drives the jaw assembly 300 to switch from the open position to the closed position and holds the jaw assembly 300 at the closed position to squeeze the tissue, and maintains the tissue in the squeezed state. When the handle 100 rebounds from the pressed position to the initial position, the connecting rod assembly 211 will not move as the handle 100 is released and rebounds, and it always remains in a state in which the jaw assembly 300 is at the closed position. When the handle 100 performs the subsequent actuation, the connecting rod assembly 211 cannot be driven to move and the state in which the jaw assembly 300 is at the closed position cannot be changed.

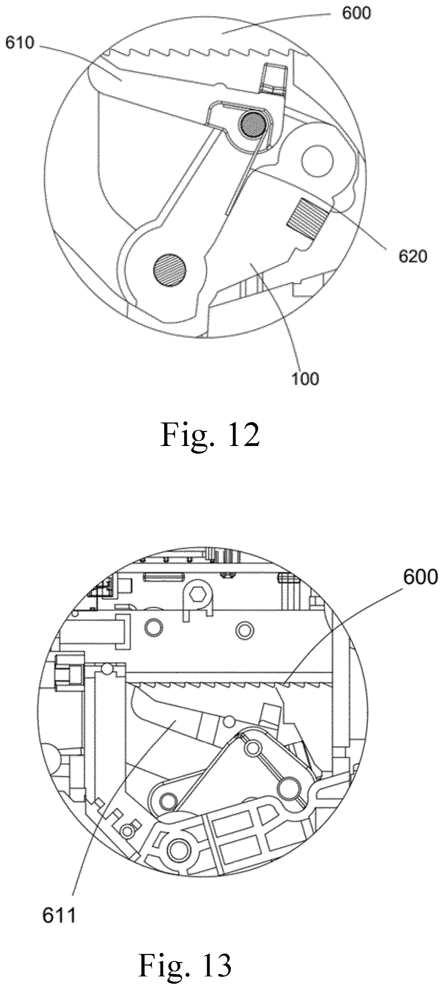

As shown in Figs. 11 and 12, the cutting knife driving member 600 is connected to the cutting knife assembly 630, and the mating member 610 is connected to the handle 100. When the mating member 610 engages with the cutting knife driving member 600, the cutting knife driving member 600 can be driven by the handle 100, so as to drive the cutting knife assembly 630.

As shown in Fig. 2, when the connecting rod assembly 211 is at the first position, the limiting member 700 restrains the mating member 610 so that the mating member 610 disengages from the cutting knife driving member 600; and thus, the first actuation of the handle 100 can only drive the jaw assembly 300 to be closed, but cannot drive the cutting knife assembly 630. As shown in Fig. 3, when the first actuation is completed and the connecting rod assembly 211 is at the second position, the limiting member 700 releases the restraint on the mating member 610, so as to enable the mating member 610 to engage with the cutting knife driving member 600. The mating member 610 being capable of engaging with the cutting knife driving member 600 means that the mating member 610 moves to a position where the mating member 610 engages with the cutting knife driving member 600. In one embodiment, the mating member 610 automatically move to engage with the cutting knife driving member 600.

In the surgical instrument provided by the embodiment of the present disclosure, only one handle 100 is provided, and the single handle 100 can realize the functions of squeezing and firing; the handle 100 can drive the jaw assembly 300 to be closed for squeezing during the first actuation, and can drive the cutting knife assembly 630 to fire during the subsequent actuation, so that the structure and operation of the surgical instrument are simpler, the weight is lighter, and the cost is lower; and the stability of the surgical instrument and the operation convenience of medical personnel are improved. The surgical instrument provided by the embodiment of the present disclosure has a simple entire structure, high reliability, and convenient operation.

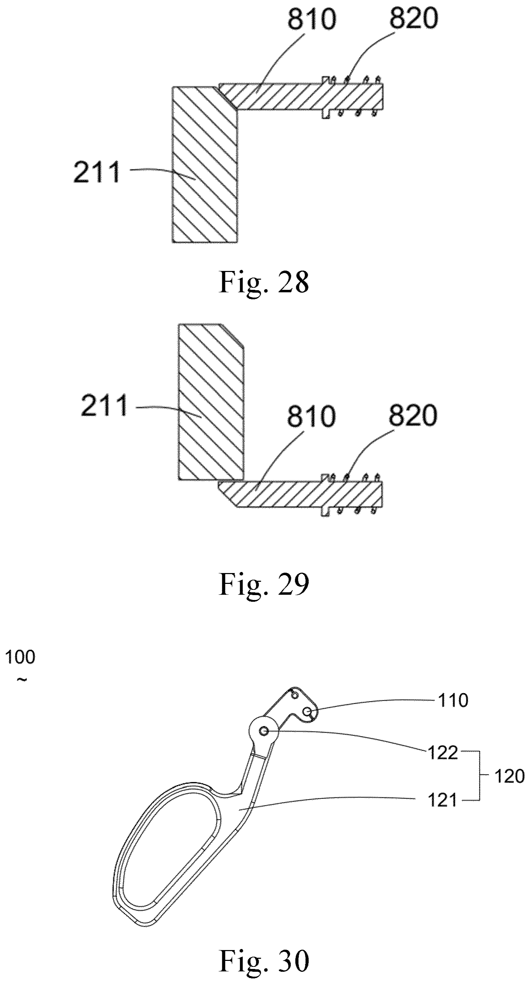

For example, as shown in Fig. 30, the handle 100 is provided with a supporting portion 110, the supporting portion 110 is located on the lower side of the connecting rod assembly 211; in the process that the connecting rod assembly 211 switches from the first position to the second position, the handle 100 supports the first connecting rod 212 or the second connecting rod 213 through the supporting portion 110, so as to be operably engaged with the connecting rod assembly 211. When the connecting rod assembly 211 is at the second position, it is locked at the second position; when the handle 100 is reset and rebounded, the supporting portion 110 detaches from the connecting rod assembly 211; when the handle 100 performs the subsequent actuation, the handle 100 switches from the initial position to the pressed position, the supporting portion 110 moves with the movement of the handle 100, the supporting portion 110 contacts the connecting rod assembly 211 at the second position only when the handle 100 reaches the pressed position (the endpoint of the movement trajectory of the supporting portion 110), that is, the supporting portion 110 does not contact the connecting rod assembly 211 during movement, and therefore, the handle 100 cannot drive the connecting rod assembly 211 during the subsequent actuation. For example, the supporting portion 110 is a rod body, and the handle 100 is operably engaged with the connecting rod assembly 211 by the supporting portion 110 abutting against the second connecting rod 213, and the supporting portion 110 can always abut against the second connecting rod 213 during the rotation of the second connecting rod 213.

As shown in Figs. 2 and 3, the limiting member 700 has a locked position and an unlocked position. When the limiting member 700 is at the locked position in Fig. 2, the limiting member 700 restrains the mating member 610 to prevent engagement between the mating member 610 and the cutting knife driving member 600. Before the handle 100 performs the first actuation, the limiting member 700 is at the locked position; in response to the first actuation of the handle 100, the connecting rod assembly 211 simultaneously drives the limiting member 700 to switch from the locked position to the unlocked position in the process that the connecting rod assembly 211 drives the jaw assembly 300 to switch from the open position to the closed position, as shown in Fig. 3; the connecting rod assembly 211 maintains the jaw assembly 300 at the closed position and also maintains the limiting member 700 at the unlocked position. At the unlocked position, the mating member 610 can engage with the cutting knife driving member 600, and during the subsequent actuation, the handle 100 can drive, through the mating member 600, the cutting knife driving member 600 to move, thereby driving the cutting knife assembly 630 to advance for firing.

As shown in Fig. 2, when being at the locked position, the limiting member 700 presses against the mating member 610 to exert a force on the mating member 610, so as to restrain the mating member 610, and thus, the mating member 610 is placed at a position where the mating member 610 cannot engage with the cutting knife driving member 600 to prevent engagement between the mating member 610 and the cutting knife driving member 600; when the limiting member 700 is at the unlocked position, the pressing against the mating member 610 is released, so that the mating member 610 can engage with the cutting knife driving member 600.

The limiting member 700 is movably arranged on the frame 410 and switches from the locked position to the unlocked position through movement. And the limiting member 700 detaches from the mating member 610 due to movement, so as to release the pressing against the mating member 610, and thus, the mating member 610 can engage with the cutting knife driving member 600. For example, the limiting member 700 is rotatably arranged on the frame 410, and the connecting rod assembly 211 can drive the limiting member 700 to rotate. The limiting member 700 keeps pressing against the mating member 610 during rotating, until the limiting member 700 rotates to the unlocked position and no longer presses against the mating member 610; that is, the limiting member 700 no longer exerts force on the mating member 610, so that the mating member 610 can engage with the cutting knife driving member 600, and can drive the cutting knife assembly 630.

For example, the limiting member 700 is substantially elongated as a whole, and includes a body portion 710 and a pressing portion 720; the pressing portion 720 is connected to the body portion 710, and for example, the pressing portion 720 and the body portion 710 are integrated; the body portion 710 is rotatably connected to the frame 410, the pressing portion 720 extends toward the mating member 610, and the limiting member 700 presses against the mating member 610 through the pressing portion 720, so as to prevent engagement between the mating member 610 and the cutting knife driving member 600. Moreover, the limiting member 700 further includes an elastic member (not shown in the figure), and the elastic member can specifically be a torsion spring. The elastic member is arranged between the body portion and the frame and is used to connect the body portion and the frame, and the elastic member exerts elastic force on the limiting member 700 to maintain the limiting member 700 at the locked position and provide a pressing force for the limiting member 700 to press against the mating member 610; when the limiting member 700 is driven by the connecting rod assembly 211 to move to the unlocked position, the elastic member is compressed. At the same time, the elastic member causes the limiting member to be at the locked position before the handle 100 performing the first actuation. The limiting member 700 further includes a protruding portion 730, and the protruding portion 730 is arranged on the body portion 710. In the process that the connecting rod assembly 211 drives the jaw assembly 300 to switch from the open position to the closed position, the connecting rod assembly 211 acts on the protruding portion 730 and drive the limiting member 700 to rotate through the protruding portion 730, and the connecting rod assembly 211 is not in contact with the body portion 710 or the pressing portion 720 to prevent interference. When the connecting rod assembly 211 maintains the jaw assembly 300 at the closed position, the connecting rod assembly 211 still abuts against the protruding portion 730, so as to maintain the limiting member 700 at the unlocked position.

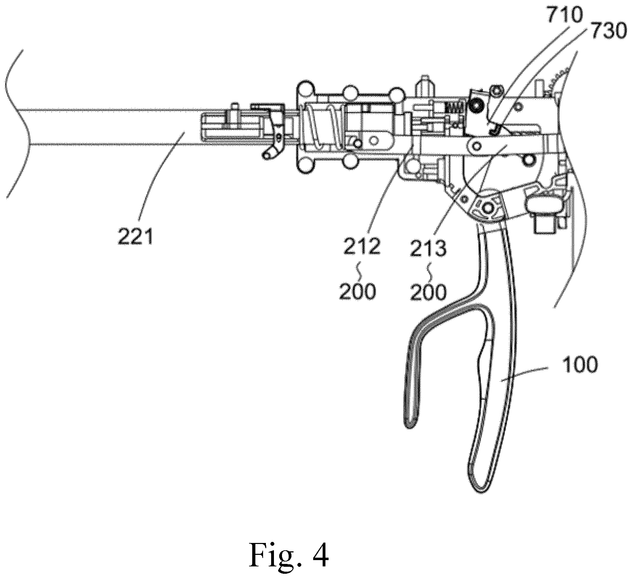

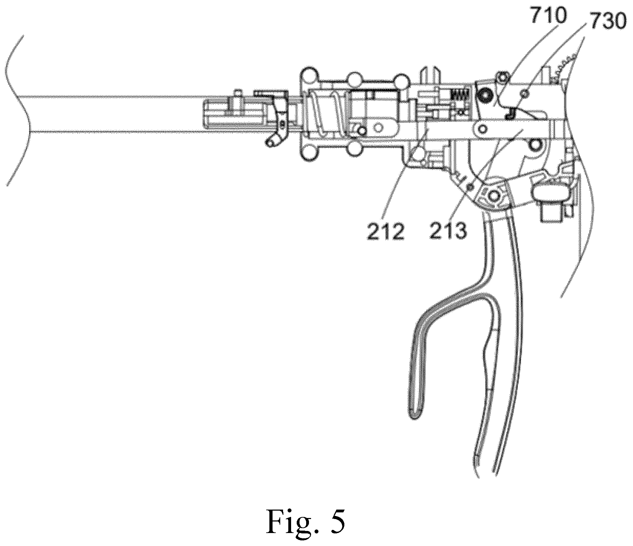

In one embodiment, as shown in Figs. 2 and 3, the protruding portion 730 is a rod body which protrudes from the body portion 710, so that the limiting member 700 can always abut against the connecting rod assembly 211 when being pushed to rotate by the connecting rod assembly 211. In another embodiment, as shown in Figs. 4 and 5, the protruding portion 730 is L-shaped and includes two sides, and a corner portion is formed between the two sides; when the protruding portion 730 contacts the connecting rod assembly 211, the corner portion of the L-shaped protruding portion 730 contacts the connecting rod assembly 211 first, and when the limiting member 700 is at the unlocked position, the protruding portion 730 abuts against the connecting rod assembly 211 through one side, so that the contact area with the connecting rod assembly 211 is increased, and further, the stability of being maintained at the unlocked position is improved. For example, the protruding portion 730 abuts against the connecting rod assembly 211 of the closing mechanism 200.

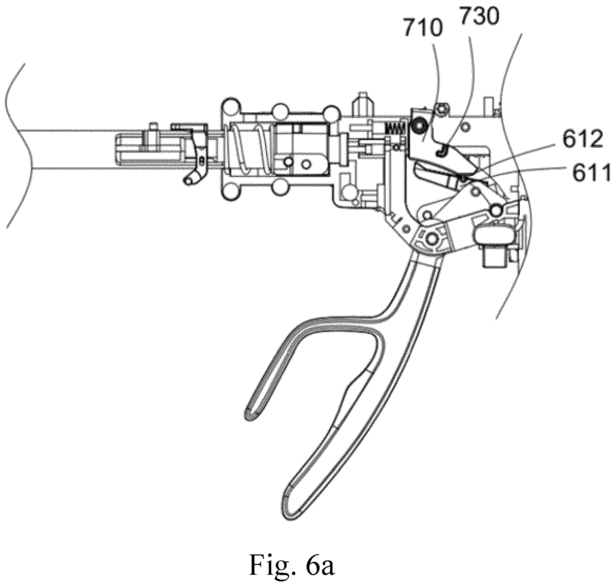

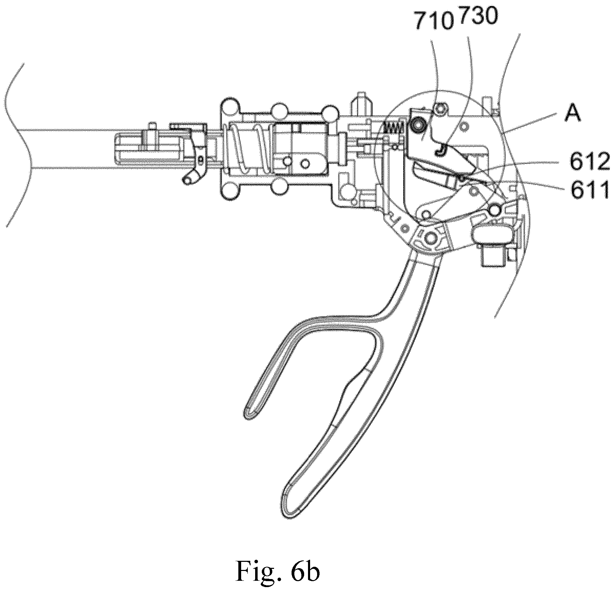

For example, when the limiting member 700 is at the unlocked position, the mating member 610 automatically move to engage with the cutting knife driving member 600, as shown in Figs. 11 and 12; the mating member 610 is movably connected to the handle 100, an elastic member 620 is provided between the handle 100 and the mating member 610, and the mating member 610 moves toward the cutting knife driving member 600 under the action of the elastic force provided by the elastic member 620, that is, the elastic force of the elastic member 620 enables the mating member 610 to move toward the direction of engaging with the cutting knife driving member 600. In the process that the limiting member 700 switches from the locked position to the unlocked position, in conjunction with Figs. 5 and 6a, when the limiting member 700 is at the locked position, the limiting member presses against the mating member 610, the elastic member 620 is compressed, and the mating member 610 cannot engage with the cutting knife driving member 600. When the limiting member 700 is at the unlocked position, the mating member 610 is no longer pressed, and under the action of the elastic force of the elastic member 620, the mating member 610 moves toward the direction of engaging with the cutting knife driving member 600, that is, when the limiting member 700 is switched to the unlocked position, the mating member 610 automatically rebounds under the action of the elastic member 620 to engage with the cutting knife driving member 600.

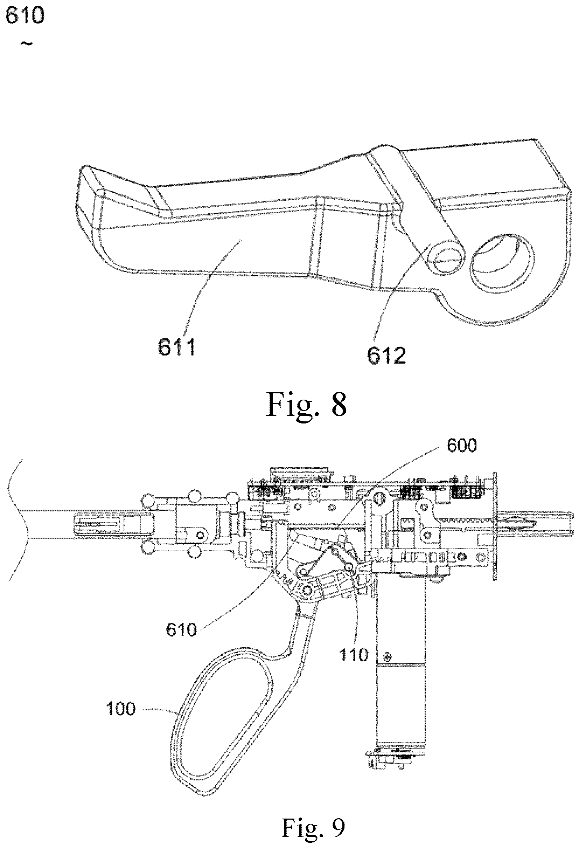

For example, as shown in Figs. 8, 9 and 12, the mating member 610 includes a pawl 611, and the cutting knife driving member 600 is a toothed member, which can specifically be a rack gear. When the mating member 610 engages with the cutting knife driving member 600, the pawl 611 meshes with the toothed member. The elastic member 620 is a torsion spring, one end of the torsion spring abuts against the handle 100, and the other end of the torsion spring abuts against the pawl 611. The elastic force provided by the torsion spring causes the pawl 611 to rotate to a position of engaging with the toothed member. When the limiting member 700 is at the locked position and presses against the pawl 611, the pawl 611 cannot engage with the toothed member, and the torsion spring is compressed. When the limiting member 700 is switched to the unlocked position, the pressing against the pawl 611 is released, and the pawl 611 rotates under the action of the torsion spring to engage with the toothed member.

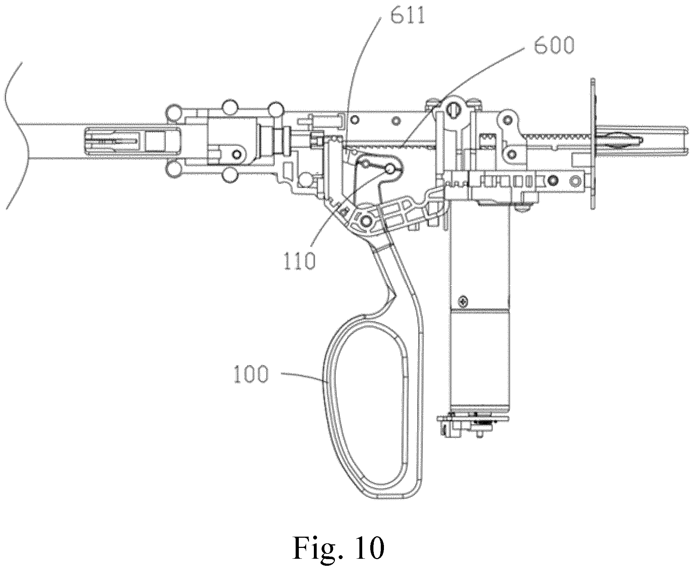

When the handle 100 performs the subsequent actuation, it can drive the pawl 611 to move through its own rotation. As shown in Figs. 9 and 10, the handle 100 is rotatably connected to the frame 410 through a hinge shaft, the pawl 611 is connected to the top end of the handle 100, and the gripping portion of the handle 100 and the pawl 611 are respectively located on both sides of the hinge shaft. When being pressed, the handle 100 rotates counterclockwise to cause the pawl 611 to move toward the distal end, thereby driving the toothed member to shift toward the distal end and cause the cutting knife assembly 630 to move toward the distal end for firing. When the handle performs the subsequent actuation, taking the second actuation as an example, when being pressed, the handle 100 drives, through the pawl 611, the toothed member and the cutting knife assembly 630 to move for firing; when being released, the handle 100 rotates clockwise back to its original position, the pawl 611 shifts toward the proximal end, the pawl 611 can only drive the toothed member in a unidirectional manner towards the distal end, the pawl 611 shifts toward the proximal end along the surface of the toothed member, while the toothed member and the cutting knife assembly 630 do not move; that is, when the handle 100 is released, the pawl 611 moves toward the proximal end and back to its original position, the toothed member and the cutting knife assembly 630 do not move, and the pawl 611 still engages with the toothed member. When performing the third actuation, the movement of the handle 100, pawl 611, toothed member, and cutting knife assembly 630 is the same as that of the second actuation. It can be seen that in the present embodiment, when the handle 100 performs the subsequent actuation, each actuation will cause the cutting knife assembly 630 to advance a certain distance, and multiple actuations can complete the entire firing of the cutting knife assembly 630.

In addition, it is to be noted that when the limiting member 700 releases the restraint on the pawl 611 and the pawl 611 moves toward the toothed member under the action of the elastic member 620, the pawl 611 has a certain chance of meshing with the toothed member, or has a certain chance of only contacting the toothed member but in a non-meshing state. As shown in Fig. 13, when the pawl 611 is in contact with but does not mesh with the toothed member, in response to the subsequent actuation of the handle 100, the pawl 611 moves toward the distal end, and the moving pawl 611 can mesh with the toothed member and drive the toothed member to move. Therefore, the pawl 611 can stably drive the toothed member to move, thereby driving the cutting knife assembly 630 to move.

Further, as shown in Figs. 6a and 8, the mating member 610 further includes an extension protrusion 612, the extension protrusion extends along the width direction of the pawl 611; the limiting member 700 presses against the mating member 610, specifically, presses against the extension protrusion 612; the setting of the extension protrusion 612 causes the position of the limiting member 700 to be offset from that of the pawl 611, so as to avoid interference between the limiting member 700 and the pawl 611.

As can be seen from the above, the first actuation of the handle 100, i.e., the process of being operated from the initial position to the pressed position for the first time, causes the connecting rod assembly 211 to switch from the first position to the second position, and the movement of the connecting rod assembly 211 causes the shift of the shaft assembly 220, which in turn causes the jaw assembly 300 to switch from the open position to the closed position; at the same time, the movement of the connecting rod assembly 211 drives the limiting member 700 to switch from the locked position to the unlocked position; when the handle 100 performs the first actuation, because of being pressed by the limiting member 700, the mating member 610 cannot engage with the cutting knife driving member 600 and cannot drive the cutting knife assembly 630. That is, the first actuation causes the jaw assembly 300 to be closed, and the cutting knife assembly 630 does not move. After completing the first actuation, the handle 100 is released, the handle 100 rebounds back to the initial position, the connecting rod assembly 211 is locked at the second position, the handle 100 detaches from the connecting rod assembly 211; during the subsequent actuation, the handle 100 is operated from the initial position to the pressed position again, the handle 100 detaches from the connecting rod assembly 211 at the second position, and only at the end of the movement path of the handle 100 will it contact the connecting rod assembly 211, thus failing to drive the connecting rod assembly 211 to move. Therefore, the handle 100 will not change the position of the connecting rod assembly 211 during the subsequent actuation, the connecting rod assembly 211 remains at the second position, and the limiting member 700 remains at the unlocked position. At the same time, during the subsequent actuation, the mating member 610 engages with the cutting knife driving member 600, and the handle 100 can drive the cutting knife driving member 600 to move by driving the mating member 610 to move, thereby driving the cutting knife assembly 630 to fire.

After the surgery, the cutting knife assembly 630 is first retracted to the initial position through a knife retracting mechanism, and then the jaw assembly is opened to release the human tissue. The closing mechanism 200 further includes an unlocking assembly (not shown in the figure), the unlocking assembly is configured to unlock the connecting rod assembly 211 at the second position; the unlocking assembly includes an operating portion arranged at the outer side of the housing of the operating assembly 400, and an unlocking portion arranged in the housing of the operating assembly and abutting against the connecting rod assembly 211 at the second position, and the unlocking portion is linked with the operating portion; when medical personnel operates the operating portion, the unlocking portion moves and pushes the connecting rod assembly 211, so that the connecting rod assembly 211 returns back to the first position, and the jaw assembly opens and releases the human tissue. For example, the operating portion can be a button provided on the housing of the operating assembly 400, and the unlocking portion is provided in the operating assembly 400 and is a rod body extending in the up-and-down direction. The button is connected to the rod body. When the button is not operated, the rod body is located above the connecting rod assembly 211, and the connecting rod assembly 211 is self-locked at the second position; when medical personnel operates the button, the rod body moves downward to abut against and push the connecting rod assembly 211 so that the connecting rod assembly 211 is no longer at the second position to release the self-locked state of the connecting rod assembly 211; and the rod body moves further downward to push the connecting rod assembly 211 to switch to the first position, thus opening the jaw assembly 300. For example, when the jaw assembly 300 is opened, the handle 100 is at the initial position, and the connecting rod assembly 211 moves under the action of the rod body, and stops after abutting against the supporting portion 110 of the handle 100; and at this time, the connecting rod assembly 211 is at the first position. When the jaw assembly 300 is in the open state, the connecting rod assembly 211 is at the first position, and the limiting member 700 returns to the locked position under the action of the elastic member.

The surgical instrument provided by the embodiment of the present disclosure is only provided with one handle 100, the handle 100 causes the jaw assembly 300 to be closed and to squeeze during the first actuation, and causes the cutting knife to advance for firing during the subsequent actuation, the entire structure is simple, and the operation is convenient. Moreover, the mating member 610 is always connected to the handle 100, and the mating member 610 can stably drive the cutting knife driving member 600, so that the firing process is more stable. The self-lock of the connecting rod assembly 211 maintains the jaw assembly 300 at the closed position, which does not require operation and does not require the setting of a locking structure, so that the entire structure of the surgical instrument is simple, the locking of the connecting rod assembly 211 is stable, and the locking failure is not easy to occur at the same time.

Another embodiment of the present disclosure provides a surgical instrument, which is substantially the same as the surgical instrument in the above embodiment, with the main difference being that the structure of the limiting member 700 is different.

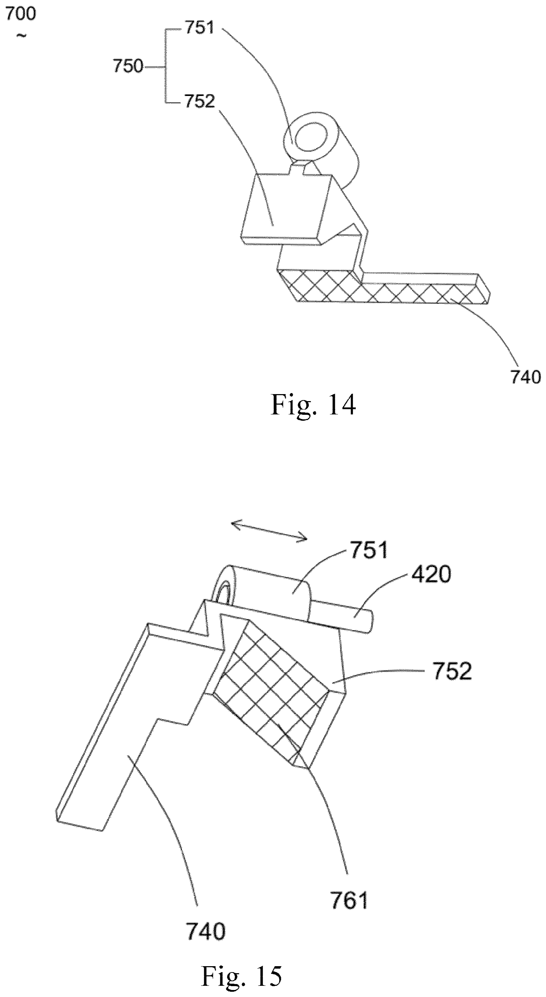

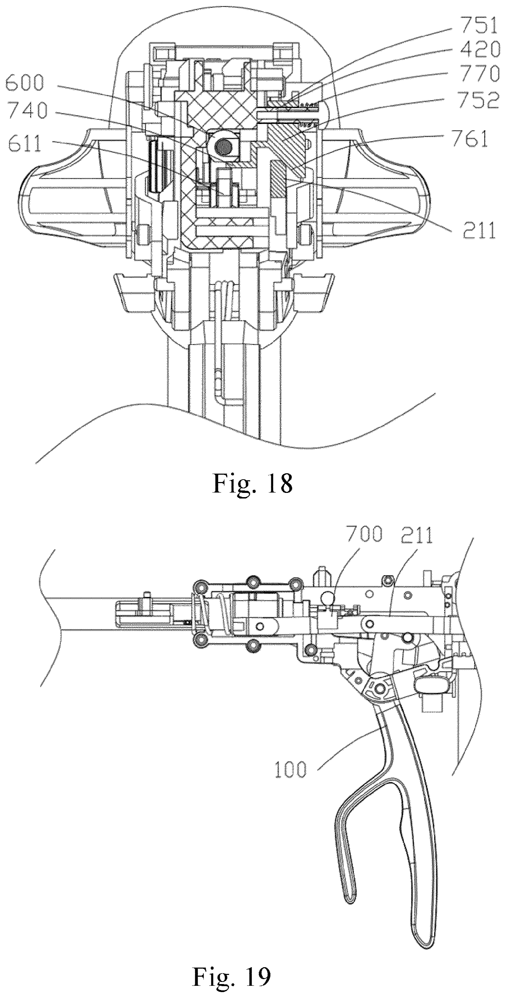

As shown in Figs. 14-20, the limiting member 700 includes a stopper 740. When the limiting member 700 is at the locked position, the stopper 740 is located between the mating member 610 and the cutting knife driving member 600, so as to separate the cutting knife driving member 600 from the mating member 610 to restrain the mating member 610, so that the mating member 610 cannot engage with the cutting knife driving member 600. When the limiting member 700 is at the unlocked position, the stopper 740 detaches from the position between the mating member 610 and the cutting knife driving member 600 to release the restraint on the mating member 610, so as to enable the mating member 610 to engage with the cutting knife driving member 600.

When being at the locked position, the stopper 740 is located between the mating member 610 and the cutting knife driving member 600, and can stably prevent the mating member 610 from engaging with the cutting knife driving member 600. During the first actuation, the mating member 610 does not engage with the cutting knife driving member 600, but still moves under the driving of the handle 100, that is, the mating member 610 slides relative to the cutting knife driving member 600; when the mating member 610 shifts under the driving of the handle, the mating member 610 always abuts against the stopper 740, and the stopper 740 has a sufficient length in the moving direction of the mating member 610, and can stably restrain the mating member 610 and prevent the mating member 610 from engaging with the cutting knife driving member.

For example, when the limiting member 700 is at the locked position, the stopper 740 abuts against at least the mating member 610 among the mating member 610 and the cutting knife driving member 600. The stopper 740 abuts against at least the mating member 610, which means that the stopper 740 only abuts against the mating member 610, or the stopper 740 simultaneously abuts against the mating member 610 and the cutting knife driving member 600. In one embodiment, one side (upper side) of the stopper 740 abuts against the cutting knife driving member 600, and the mating member 610 abuts against the other side (lower side) of the stopper 740 under the action of the elastic member 620. In another embodiment, the upper side of the stopper 740 detaches from the cutting knife driving member 600, and the lower side of the stopper 740 abuts against the mating member 610.

As shown in Fig. 14, the limiting member 700 further includes a movement portion 750, the movement portion 750 is movably arranged on the frame 410, and the movement portion 750 is connected to the stopper 740, and can drive the stopper 740 to move, so that the stopper 740 detaches from the position between the mating member 610 and the cutting knife driving member 600. In the process that the closing mechanism 200 drives the jaw assembly 300 to move from the open position to the closed position, the closing mechanism 200 drives the movement portion 750 to move, thereby driving the limiting member 700 to switch from the locked position to the unlocked position. Specifically, in the process that the connecting rod assembly 211 switches from the first position to the second position, the connecting rod assembly 211 drives the movement portion 750 to move, and the movement portion 750 drives the stopper 740 to move.

The movement portion 750 is slidably arranged on the frame 410. As shown in Fig. 14, the movement portion 750 includes a sliding portion 751 and a guide portion 752, the sliding portion 751 is slidably connected to the frame 410, the guide portion 752 is connected to the sliding portion 751, the guide portion 752 has a guide inclined surface 761 (as shown in Fig. 15); in the process that the connecting rod assembly 211 switches from the first position to the second position, the connecting rod assembly 211 abuts against and pushes the guide portion 752 to drive the movement portion 750 to move, so as to drive the stopper 740 to detach from the position between the mating member 610 and the cutting knife driving member 600. For example, as shown in Figs. 15, 17 and 18, the frame 410 is provided with a sliding shaft 420, the sliding portion 751 is arranged in a ring shape and is sleeved around the sliding shaft 420, the sliding portion 751 can move along the axial direction of the sliding shaft 420; the movement portion 750 further includes a spring 770, the spring 770 is arranged on one side of the sliding portion 751 away from the stopper 740 and is sleeved on the sliding shaft 420 for exerting elastic force on the sliding portion 751. A screw 780 is provided at one end of the sliding shaft 420 away from the frame 410, the screw 780 includes a stud and a nut 782; the stud is screwed into the sliding shaft 420 to engage with the sliding shaft 420, the nut 782 is located on one side of the sliding shaft 420 away from the frame 410, one end of the spring 770 abuts against the nut 782, and the other end of the spring 770 abuts against the sliding portion 751. The guide portion 752 is located below the movement portion 750, the guide inclined surface 761 has a high side and a low side from top to bottom, the high side is located between the low side and the stopper 740 in the front-rear direction; in the process of switching from the first position to the second position, the connecting rod assembly 211 gradually moves upward, firstly abutting against the low side of the guide inclined surface 761, and then pushing the guide portion 752 to move until abutting against the high side of the guide inclined surface 761; in the process of being pushed by the connecting rod assembly 211, the guide portion 752 drives the sliding portion 751 to move in a direction away from the mating member 610 relative to the sliding shaft 420, thereby driving the stopper 740 to move to detach from the position between the mating member 610 and the cutting knife driving member 600. And in this process, the spring 770 is compressed.

For example, the first connecting rod 212 and/or the second connecting rod 213 in the connecting rod assembly 211 is provided with a mating inclined surface 762. Specifically, the connecting rod contacting the guide portion 752 in the process that the connecting rod assembly 211 switches from the first position to the second position is provided with a mating inclined surface 762. The slope of the mating inclined surface 762 is substantially consistent with the slope of the guide inclined surface 761, so as to better push the guide portion 752 to move.

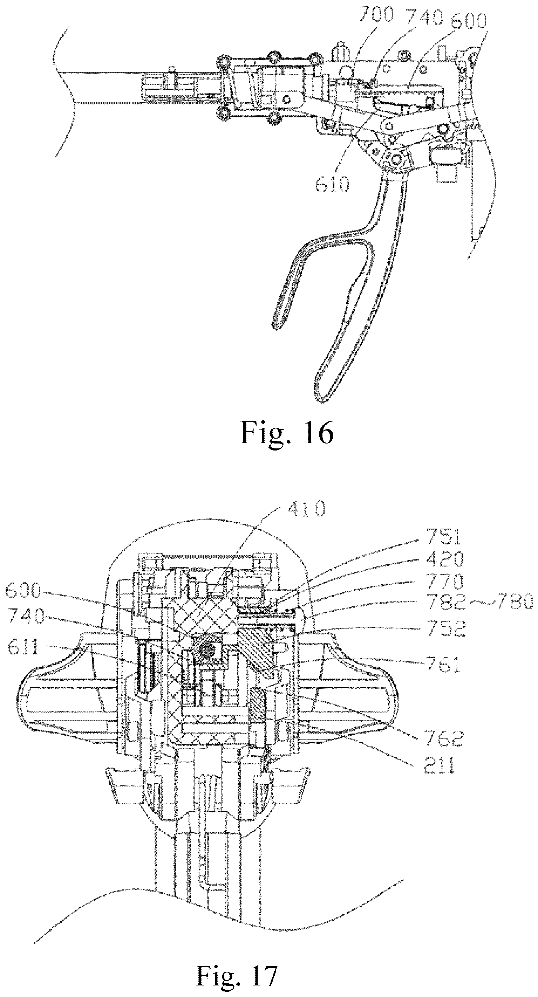

For example, as shown in Figs. 16 and 17, the stopper 740 is provided in a sheet shape, and the width of the stopper 740 is substantially the same as the width of the cutting knife driving member 600, so as to cover the surface of the cutting knife driving member 600 and prevent the mating member 610 and the cutting knife driving member 600 while saving the material of the stopper 740 and facilitating the movement of the limiting member 700.

An embodiment of the present disclosure provides a surgical instrument, which is substantially the same as the surgical instrument in the above embodiments, with the main difference being that the structure of the limiting member 700 is different.

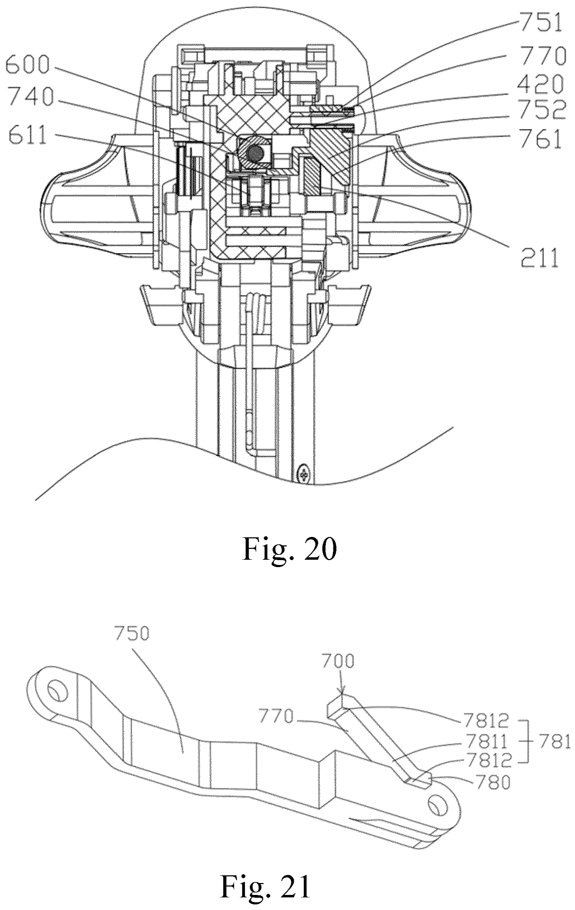

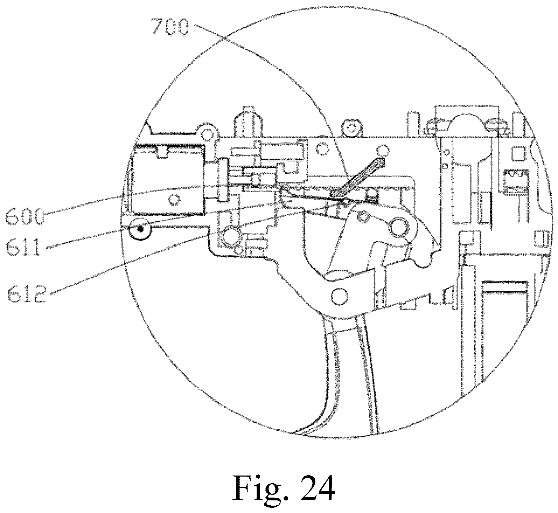

As shown in Figs. 21-24, the limiting member 700 is arranged on the first connecting rod 212 or the second connecting rod 213; in the process that the connecting rod assembly 211 switches from the first position to the second position, the limiting member 700 moves with the connecting rod assembly 211, and switches from the locked position to the unlocked position, so as to release the restraint on the mating member 610. The limiting member 700 moves with the connecting rod assembly 211 to switch from the locked position to the unlocked position, and the position switching is more stable.

For example, because the second connecting rod 213 is close to the mating member 610, the limiting member 700 is a rod body provided on the second connecting rod 213 and forms an angle with the second connecting rod 213; and the limiting member 700 and the second connecting rod 213 form an acute angle with each other, so that the limiting member 700 is obliquely provided on the second connecting rod 213. The limiting member 700 is partially offset from the second connecting rod 213, and the limiting member 700 includes a body portion 770 and a blocking portion 780, the body portion 770 is connected to the first connecting rod 212 or the second connecting rod 213, and the projection of the body portion 770 in the vertical direction falls on the first connecting rod 212 or the second connecting rod 213; the blocking portion 780 is connected to the body portion 770, for example, integrally formed with the body portion 770, and protrudes toward the mating member 610. The projection of the mating member 610 in the vertical direction does not fall on the first connecting rod 212 or the second connecting rod 213, so as to avoid interference with the moving connecting rod assembly 211.

As shown in Fig. 21, the overall shape of the limiting member 700 is obliquely arranged, and the front end and the rear end thereof are both arranged in a flat plate shape. The blocking portion 780 has an abutting surface 781, the abutting surface 781 specifically includes an inclined surface 7811 and two flat surfaces 7812 connected to the front end and the rear end of the inclined surface 7811. When the connecting rod assembly 211 is at the first position, the limiting member 700 is at the locked position, the limiting member 700 abuts against the extension protrusion 612 of the mating member 610 through the abutting surface 781, specifically, abuts against the extension protrusion 612 through the inclined surface 7811; the extension protrusion 612 is arranged in a column shape and can move along the inclined surface 7811; under the action of the elastic member 620, the mating member 610 has a tendency to move toward the cutting knife driving member 600 (toward the front end), and the flat surface 7812 connected to the front end of the inclined surface 7811 can prevent the mating member 610 from detaching from the abutting surface 781.

In the process that the connecting rod assembly 211 moves from the first position to the second position, it drives the limiting member 700 move upward and rotate, so that the extension protrusion 612 of the mating member 610 moves relative to the abutting surface 781. Specifically, the extension protrusion 612 moves toward the rear end along the inclined surface 7811 and detaches from the flat surface 7812 at the rear end. In this process, the elastic member 620 is gradually released. After the connecting rod assembly 211 is at the second position, the limiting member 700 is entirely located above the extension protrusion 612, the elastic member 620 is completely released, the mating member 610 engages with the cutting knife driving member 600 and detaches from the limiting member 700, and the limiting member 700 will not prevent the mating member 610 from subsequently driving the cutting knife driving member 600.

In common technology, the operating assembly is generally provided with two handles to respectively control jaw closure and cutting knife advancement, resulting in a complex structure and cumbersome operation.

In common technology, there exists a stapler with a single handle that can control jaw closure and cutting knife advancement, which includes a handle, a cam, a connecting rod, a rack gear, and a ratchet assembly. The handle is connected to the cam. Before the handle performs a first actuation, the ratchet assembly disengages from the handle, and the ratchet assembly disengages from the rack gear. When the handle performs the first actuation, the cam rotates and drives the connecting rod to move to close the jaws. After rotation, the cam is locked by a spring mechanism. When the cam rotates, the cam creates space for the ratchet assembly to move. After movement, the ratchet assembly meshes with the handle and the rack gear simultaneously, allowing the handle to drive the jaw rack gear during a subsequent actuation, and thus driving the cutting knife. However, the ratchet assembly moves while engaging with the handle and the rack gear, which has a poor stability, may easily cause the handle to get stuck, and prevent the surgery from proceeding normally.

An embodiment of the present disclosure provides a surgical instrument, which is capable of controlling jaw closure and cutting knife advancement (firing) through a single handle, has a simple structure and is easy to operate; and the handle will not get stuck and has high stability.

As shown in Figs. 1, 2, 3 and 11, an embodiment of the present disclosure provides a surgical instrument, which includes an operating assembly 400, and a jaw assembly 300 and a cutting knife assembly 630 respectively connected to the operating assembly 400; the operating assembly 400 includes: a handle 100, operable to perform a first actuation and a subsequent actuation; a closing mechanism 200, connected to the jaw assembly 300; a cutting knife driving member 600, connected to the cutting knife assembly 630; a mating member 610, connected to the handle 100; a limiting member 700, having a locked position and an unlocked position; when being at the locked position, the limiting member 700 restrains the mating member 610 to prevent engagement between the mating member 610 and the cutting knife driving member 600; in response to the first actuation of the handle 100, the closing mechanism 200 drives the jaw assembly 300 to switch from an open position to a closed position and holds the jaw assembly 300 at the closed position, and drives the limiting member 700 to switch from the locked position to the unlocked position; when the jaw assembly 300 is at the closed position, the limiting member 700 is at the unlocked position; when the limiting member 700 is at the unlocked position, the limiting member 700 releases a restraint on the mating member 610 to enable the mating member 610 to engage with the cutting knife driving member 600, and in response to the subsequent actuation of the handle 100, the mating member 610 drives the cutting knife driving member 600 to move, so as to drive the cutting knife assembly 630.

As shown in Figs. 2 and 3, the limiting member 700 has a locked position and an unlocked position. When the limiting member 700 is at the locked position in Fig. 2, the limiting member 700 restrains the mating member 610 to prevent engagement between the mating member 610 and the cutting knife driving member 600. Before the handle 100 performs the first actuation, the limiting member 700 is at the locked position; in response to the first actuation of the handle 100, the closing mechanism 200 simultaneously drives the limiting member 700 to switch from the locked position to the unlocked position in the process that the closing mechanism 200 drives the jaw assembly 300 to switch from the open position to the closed position. As shown in Fig. 3, the closing mechanism 200 maintains the jaw assembly 300 at the closed position and also maintains the limiting member 700 at the unlocked position. At the unlocked position, the mating member 610 can engage with the cutting knife driving member 600, and during the subsequent actuation, the handle 100 can drive, through the mating member 600, the cutting knife driving member 600 to move, thereby driving the cutting knife assembly 630 to advance for firing.

In the surgical instrument of the present embodiment, only one handle 100 is provided, and the single handle 100 can realize the functions of squeezing and firing; the handle 100 can drive the jaw assembly 300 to be closed for squeezing during the first actuation, and can drive the cutting knife assembly 630 to fire during the subsequent actuation, so that the structure and operation of the surgical instrument are simpler, the stability is high, and the stability of the surgical instrument and the convenience of operation by medical personnel are improved.

Before the first actuation of the handle 100, the limiting member 700 is at the locked position, and the pressing portion 720 presses against the extension protrusion 612. When the handle 100 performs the first actuation, the limiting member 700 rotates counterclockwise, the pressing portion 720 rotates synchronously with the limiting member 7001, the pawl 611 rotates clockwise to bring the tip of the pawl 611 closer to the toothed member, and the pressing portion 720 always presses against the extension protrusion 612; after the limiting member 700 reaches the unlocked position, the pawl 611 abuts against the toothed member, the pressing portion 720 is located above the extension protrusion 612 and no longer presses against the extension protrusion 612, so that the pawl 611 can drive the toothed member to move.

In the present embodiment, as shown in Fig. 2, the closing mechanism 200 includes a shaft driving member 210 and a shaft assembly 220, the shaft assembly 220 is connected to the jaw assembly 300, and the shaft driving member 210 is connected to the shaft assembly 220. When the handle 100 performs the first actuation, the handle 100 is operably engaged with the shaft driving member 210 to enable the handle 100 to drive the shaft driving member 210 to move, and when the shaft driving member 210 moves, it can drive the shaft assembly 220 to move, so as to cause the jaw assembly 300 to switch from the open position to the closed position. Specifically, the shaft assembly 220 includes an inner cannula 222 and an outer cannula 221, the outer cannula 221 is looped around the inner cannula 222, and the outer cannula 221 can move relative to the inner cannula 222; one end of the outer cannula 221 is connected to the shaft driving member 210, and the other end of the outer cannula 221 is connected to the jaw assembly 300. In response to the first actuation of the handle 100, the shaft driving member 210 switches from the first position to the second position to drive the outer cannula 221 to move, and the movement of the outer cannula 221 causes the jaw assembly 300 to switch from the open position to the closed position. When the handle 100 is switched to the pressed position, the shaft driving member 210 is locked at the second position, which in turn locks the position of the outer cannula 221, and the jaw assembly 300 remains at the closed position. When the handle 100 rebounds from the pressed position to the initial position, the shaft driving member 210 remains at the second position and does not move with the handle 100, and the outer cannula 221 also remains at the position that causes the jaw assembly 300 to be at the closed position.

As shown in Figs. 2 and 3, the shaft driving member 210 includes a connecting rod assembly 211, the connecting rod assembly includes a first connecting rod 212 and a second connecting rod 213; the first connecting rod 212 is rotatably connected to the shaft assembly 220, specifically to the outer cannula 221. One end of the second connecting rod 213 is rotatably connected to the frame 410, and the other end of the second connecting rod 213 is rotatably connected to the first connecting rod 212, so as to form a dual-link mechanism. When the connecting rod assembly 211 is at the first position, the first connecting rod 212 and the second connecting rod 213 form an angle with each other, and both the first connecting rod 212 and the second connecting rod 213 are inclined toward the lower side (a side close to the gripping portion of the handle 100). When the connecting rod assembly 211 is at the first position and the handle 100 has not performed the first actuation, the handle 100 is in contact with the first connecting rod 212 or the second connecting rod 213 to enable the handle 100 to drive the connecting rod assembly 211 to move, the distal end of the first connecting rod 212 is rotatably connected to the outer cannula 221, the proximal end of the first connecting rod 212 is rotatably connected to the second connecting rod 213, and the first connecting rod 212 is rotatably connected to the second connecting rod 213 through a hinge point. In the process that the connecting rod assembly 211 switches from the first position to the second position, the hinge point gradually moves to the upper side (a side away from the gripping portion of the handle 100), and because the proximal end of the second connecting rod 213 is connected to the frame 410, the hinge point located at the distal end of the second connecting rod 213 moves toward the distal end; at the same time, the rotation of the first connecting rod 212 causes the distal end of the first connecting rod 212 to move toward the distal end, and therefore, the connecting rod assembly 211 can drive the outer cannula 221 to move toward the distal end, so as to close the jaw assembly 300.

As shown in Fig. 3, when the connecting rod assembly 211 is at the second position, the connecting rod assembly 211 is at or substantially at the dead center. Being at the dead center means that the first connecting rod 212 and the second connecting rod 213 are located on a same straight line, and an included angle between the first connecting rod 212 and the second connecting rod 213 is 180 degrees. Being substantially at the dead center means that the first connecting rod 212 and the second connecting rod 213 go beyond a dead center position, and the included angle between the first connecting rod 212 and the second connecting rod 213 is greater than 0 degree and less than 5 degrees. When the connecting rod assembly 211 is at the dead center or substantially at the dead center, the pressure angle between the first connecting rod 212 and the second connecting rod 213 is substantially equal to 90 degrees, and when the first connecting rod 212 or the second connecting rod 213 is subjected to external force, the torque exerted on the other connecting rod is zero, so that the connecting rod assembly 211 cannot move, and the connecting rod assembly 211 is self-locked at the second position.

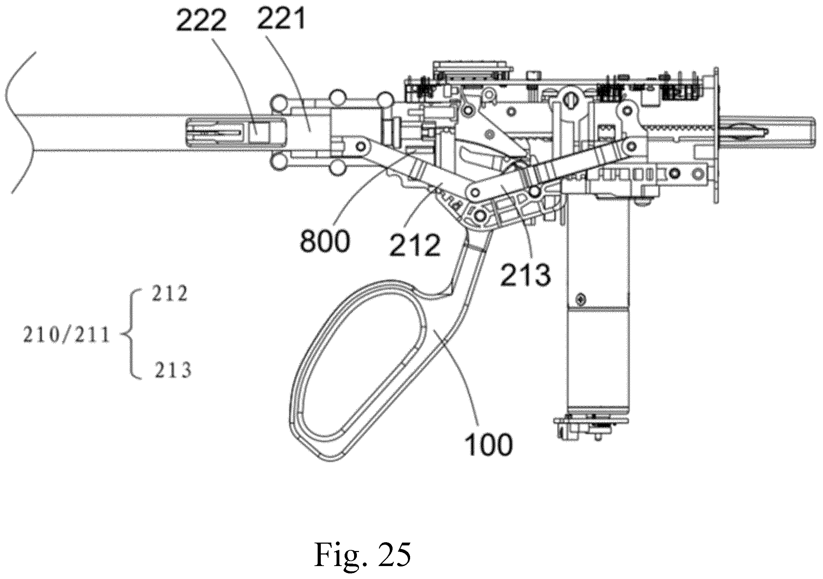

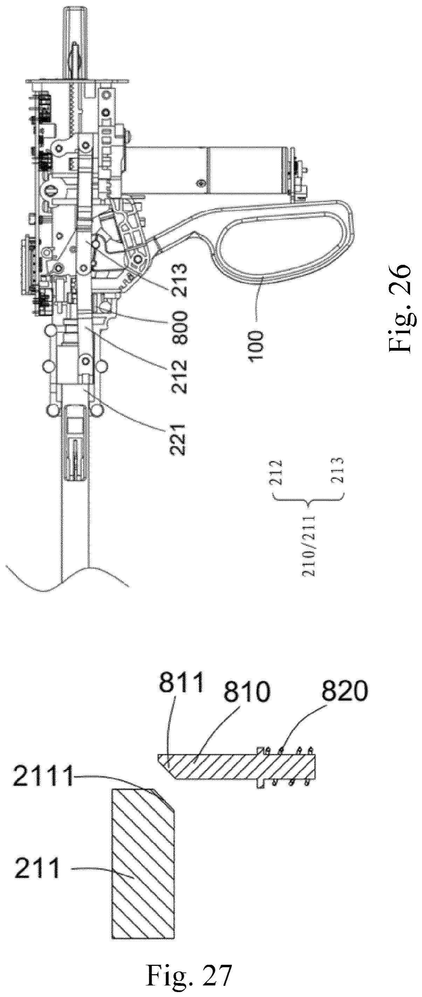

In one embodiment, as shown in Figs. 25-29, the closing mechanism 200 further includes a locking member 800; when the shaft driving member 210 is at the second position, the locking member 800 engages with the shaft driving member 210 to lock the shaft driving member 210 at the second position, so that the jaw assembly 300 remains at the closed position. For example, the locking member 800 is a movable spring pin assembly including a spring member 820 and a pin 810. The spring member 820 is sleeved on the pin 810, the pin 810 is provided with a protrusion, one end of the spring member 820 abuts against the protrusion of the pin 810, and the other end of the spring member 820 abuts against the frame. When the connecting rod assembly 211 is at the first position, the pin 810 is located above the connecting rod assembly 211; and when the connecting rod assembly 211 is at the second position, the pin 810 is located below the connecting rod assembly 211. That is, during switching from the first position to the second position, the connecting rod assembly 211 passes over the pin 810, the pin 810 above the connecting rod assembly 211 blocks the connecting rod assembly 211; a guide surface is provided at the lower side of the pin 810, and in the process that the connecting rod assembly 211 passes over the pin 810, the connecting rod assembly 211 first abuts against the guide surface 811 of the pin 810; the guide surface 811 is an inclined surface, and the connecting rod assembly 211 abuts against the guide surface 811, so that the pin 810 can move relative to the connecting rod assembly 211. For example, a mating inclined surface 2111 is provided at the upper side of the connecting rod assembly 211; when the connecting rod assembly 211 moves toward the second position (upward), the pin 810 retracts under the action of the mating inclined surface 2111 and the guide surface 811 to create space for the connecting rod assembly 211, compress the spring member 820, and no longer blocks the connecting rod assembly 211, so that the connecting rod assembly 211 can pass over the pin 810. When switched to the second position, the connecting rod assembly 211 detaches from the pin 810; the pin 810 rebounds under the action of the spring member 820, protrudes below the connecting rod assembly 211, and abuts against the connecting rod assembly 211 from below the connecting rod assembly 211, so as to engage with the connecting rod assembly 211 and prevent the connecting rod assembly 211 from moving to the first position (downward), thus locking the connecting rod assembly 211 at the second position. For example, an operating member capable of retracting the pin 810 is provided on the housing of the operating assembly, the operating member is connected to the pin 810, pulling the operating member to cause the pin 810 to retract, and the spring is compressed. When opening the jaw assembly 300, medical personnel pulls the operating member to retract the pin 810, so that the pin 810 no longer abuts against the connecting rod assembly 211 from below, and the mating between the pin 810 and the connecting rod assembly 211 is disabled; and therefore, the connecting rod assembly 211 can move toward the first position, and the jaw assembly 300 can be opened. Of course, in other embodiments, the locking member 800 can have other configurations, and the connecting rod assembly 211 can be locked in other ways, which are not specifically limited in the embodiments of the present disclosure. The locking member can be used in combination with self-locking of the connecting rod assembly 211 at the dead center or can be used independently.

Further, as shown in Fig. 30, the handle 100 is provided with a supporting portion 110, the supporting portion 110 is located on the lower side of the connecting rod assembly 211. In the process that the connecting rod assembly 211 switches from the first position to the second position, the handle 100 supports the first connecting rod 212 or the second connecting rod 213 through the supporting portion 110, so as to be operably engaged with the connecting rod assembly 211. When the connecting rod assembly 211 is at the second position, it is locked at the second position; when the handle 100 is reset and rebounded, the supporting portion 110 detaches from the connecting rod assembly 211. When the handle 100 performs the subsequent actuation, the handle 100 switches from the initial position to the pressed position, the supporting portion 110 moves with the movement of the handle 100, the supporting portion 110 contacts the connecting rod assembly 211 at the second position only when the handle 100 reaches the pressed position (the endpoint of the movement trajectory of the supporting portion 110). That is, the supporting portion 110 does not contact the connecting rod assembly 211 during movement, and therefore, the handle 100 cannot drive the connecting rod assembly 211 during the subsequent actuation. Specifically, the supporting portion 110 is a rod body, and the handle 100 is operably engaged with the connecting rod assembly 211 by the supporting portion 110 abutting against the second connecting rod 213, and the supporting portion 110 can always abut against the second connecting rod 213 during the rotation of the second connecting rod 213.

The surgical instrument provided by the embodiment of the present disclosure is only provided with one handle, the handle causes the jaw assembly to be closed and to squeeze during the first actuation, and causes the cutting knife to advance for firing during the subsequent actuation, the entire structure is simple, the reliability is high, and the operation is simple and convenient. Moreover, the mating member is always connected to the handle, and the mating member can stably drive the cutting knife driving member, so that the firing process is more stable.

In common technology, a connecting rod assembly is arranged in the operating assembly, the connecting rod assembly is connected to an outer cannula, and the handle drives the connecting rod assembly to move to drive the outer cannula to move, and then drives the jaw assembly to close the jaw assembly, but the overall stability of the connecting rod assembly driving the jaw assembly to be closed is poor, which can easily lead to unstable jaw assembly closure and unstable tissue squeeze.

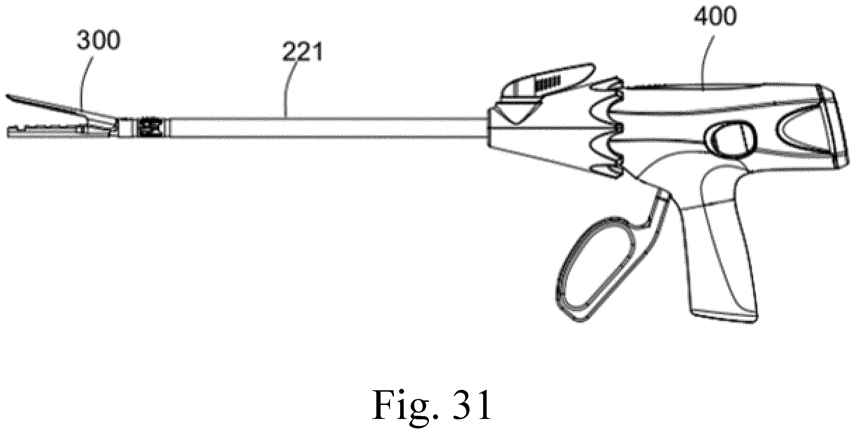

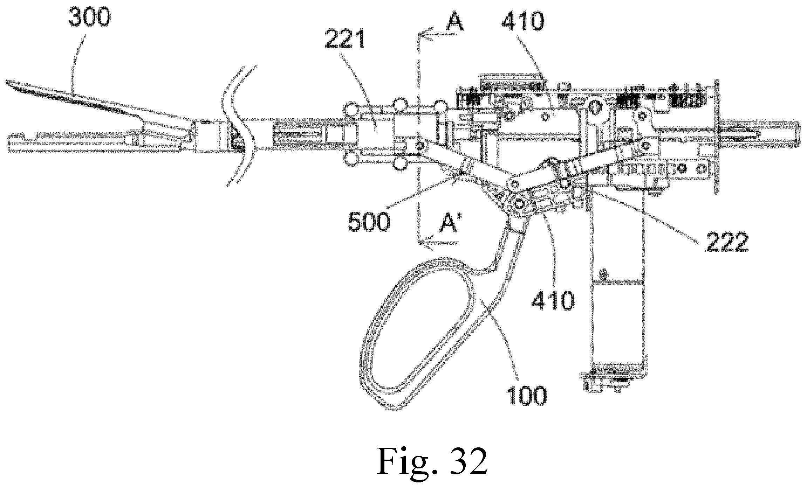

An embodiment of the present disclosure provides a surgical instrument, which can be a stapler, as shown in Figs. 31 and 32, the surgical instrument includes an operating assembly 400 and a jaw assembly 300 connected to the operating assembly 400. The operating assembly 400 includes a frame 410, a handle 100 and a connecting rod assembly 500, the handle 100 is rotatably connected to the frame 410, an elastic assembly (not shown in the figure) is arranged between the handle 100 and the frame 410, and the handle 100 has an initial position and a pressed position. The medical personnel can cause the handle 100 to move from the initial position to the pressed position by pressing the handle 100. After the handle 100 is released, the handle 100 can rebound from the pressed position to the initial position under the action of the elastic assembly. The process of the handle 100 moving from the initial position to the pressed position is called actuation. During surgery, the handle 100 is operated to perform an actuation, and when the handle 100 performs an actuation, the connecting rod assembly 500 is driven to move, and in response to the movement of the connecting rod assembly 500, the jaw assembly 300 moves from the open position to the closed position.

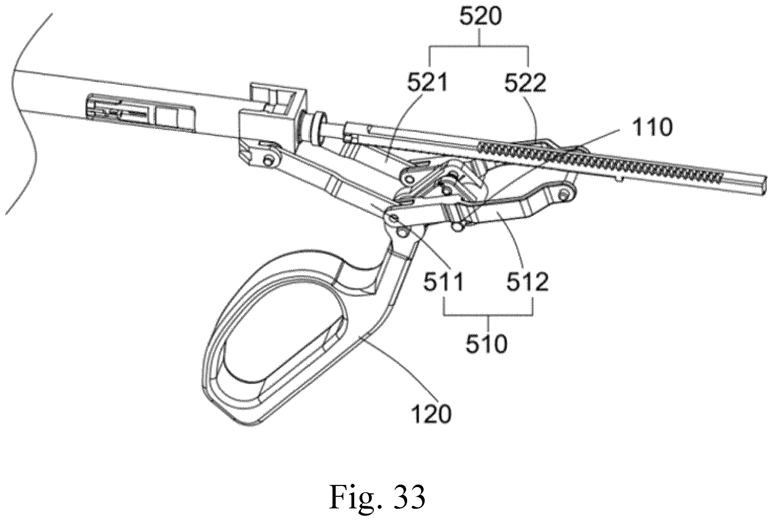

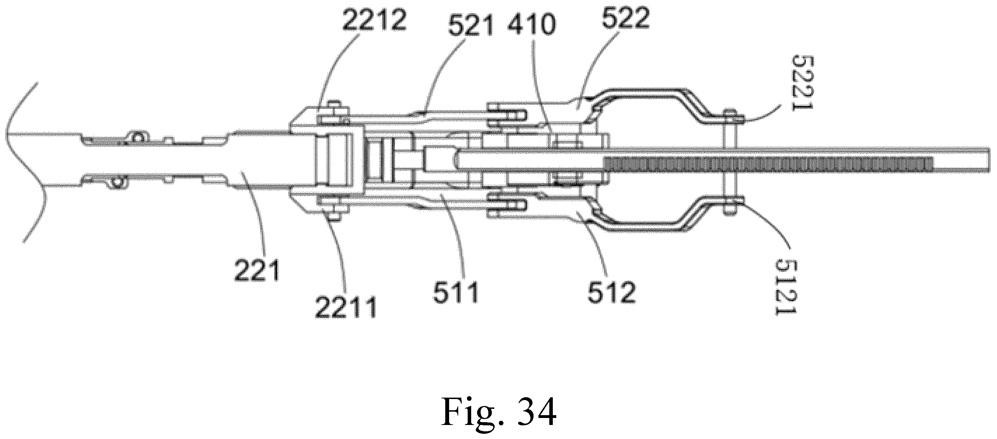

For example, the connecting rod assembly 500 is operably engaged with the handle 100, and the handle 100 is capable of driving the connecting rod assembly 500 to move during actuation. For example, as shown in Figs. 33 and 34, the connecting rod assembly 500 includes a left connecting rod 510 and a right connecting rod 520, and the left connecting rod 510 and the right connecting rod 520 are located on both sides of the frame 410, respectively. That is, the left connecting rod 510 is located on one side of the frame 410, and the right connecting rod 520 is located on the other side of the frame 410. The left connecting rod 510 is connected to the right connecting rod 520, and the handle 100 is operably engaged with the left connecting rod 510 and/or the right connecting rod 520, so that the handle 100, upon actuation, can simultaneously drive the left connecting rod 510 and the right connecting rod 520, thus driving the connecting rod assembly 500 and causing the jaw assembly 300 to move from the open position to the closed position. For example, the left connecting rod 510 and the right connecting rod 520 are connected and can move synchronously under the driving of the handle 100, so that the movement of the connecting rod assembly 500 is more stable, and further, the handle 100 can drive the jaw assembly 300 to be closed more stably.

For example, the left connecting rod 510 includes a left first connecting rod 511 and a left second connecting rod 512, the left first connecting rod 511 is rotatably connected to the jaw assembly 300, one end of the left second connecting rod 512 is rotatably connected to the frame 410, and the other end of the left second connecting rod 512 is rotatably connected to the left first connecting rod 511; the right connecting rod 520 includes a right first connecting rod 521 and a right second connecting rod 522, the right first connecting rod 521 is rotatably connected to the jaw assembly 300, one end of the right second connecting rod 522 is rotatably connected to the frame 410, and the other end of the right second connecting rod 522 is connected to the right first connecting rod 521. The left second connecting rod 512 is connected to the right second connecting rod 522, so that the left connecting rod 510 can move synchronously with the right connecting rod 520. For example, at least one of the left first connecting rod 511, the left second connecting rod 512, the right first connecting rod 521, and the right second connecting rod 522 can be operably engaged with the handle 100, so that the handle 100 is operably engaged with the left connecting rod 510 and/or the right connecting rod 520.

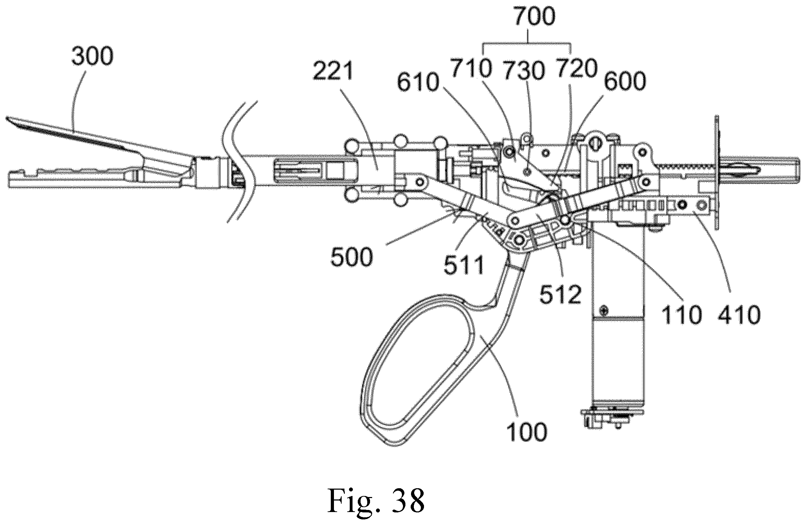

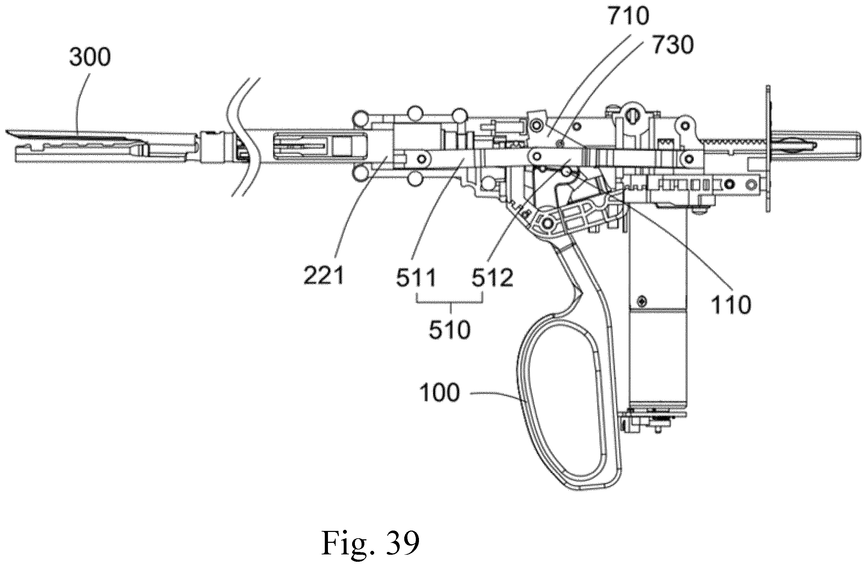

For example, the handle 100, upon actuation, drives the left connecting rod 510 and/or the right connecting rod 520 to move, and the left connecting rod 510 and the right connecting rod 520 move synchronously and in a mirrored way. When describing the state of the connecting rod assembly 500, only the left connecting rod 510 is taken as an example for illustration in the present embodiment, and the state of the right connecting rod 520 is the same as that of the left connecting rod 510. As shown in Fig. 38, when the handle 100 is at the initial position, the connecting rod assembly 500 is at the first position. As shown in Fig. 39, when the handle 100 moves to the pressed position, the connecting rod assembly 500 is at the second position. When the connecting rod assembly 500 is at the first position, the left first connecting rod 511 and the left second connecting rod 512 form an angle with each other, and both of the left first connecting rod 511 and the left second connecting rod 512 are inclined and are in a substantially V-shape toward the gripping portion 121 of the handle 100 (as shown in Fig. 30). As shown in Fig. 39, when the connecting rod assembly 500 is at the second position, the left connecting rod 510 is at or substantially at the dead center, and at the same time, the right connecting rod 520 is at or substantially at the dead center. Being at the dead center means that the left first connecting rod 511 and the left second connecting rod 512 are located on a same straight line, and an included angle between the left first connecting rod 511 and the left second connecting rod 512 is 0 degree (or 180 degrees). Being substantially at the dead center means that the left first connecting rod 511 and the left second connecting rod 512 go beyond a dead center position, and the included angle between the left first connecting rod 511 and the left second connecting rod 512 is greater than 0 degree and less than 5 degrees. When the left connecting rod 510 is at the dead center position or substantially at the dead center position, the pressure angle between the left first connecting rod 511 and the left second connecting rod 512 is substantially equal to 90 degrees, and when the left first connecting rod 511 or the left second connecting rod 512 is subjected to external force, the torque exerted on the other connecting rod is zero, so that the left connecting rod 510 cannot move, and the left connecting rod 510 is locked at the second position. When the left connecting rod 510 is at the dead center, the right connecting rod 520 is also at the dead center; when the left connecting rod 510 is substantially at the dead center, the right connecting rod 520 is substantially at the dead center; and the right connecting rod 520 and left connecting rod 510 are synchronously locked at the second positions, so that the connecting rod assembly 500 is self-locked at the second position. The left connecting rod 510 and the right connecting rod 520 are synchronously locked, thus improving the self-locking stability of the connecting rod assembly 500.

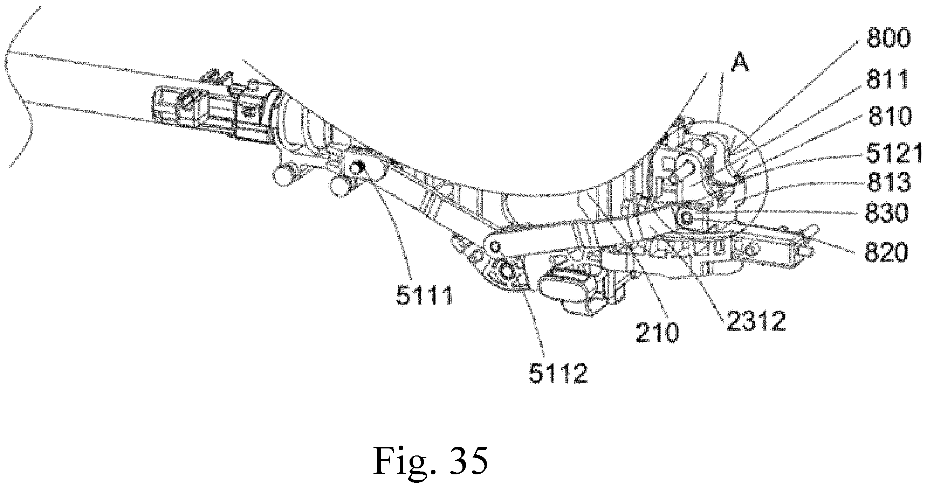

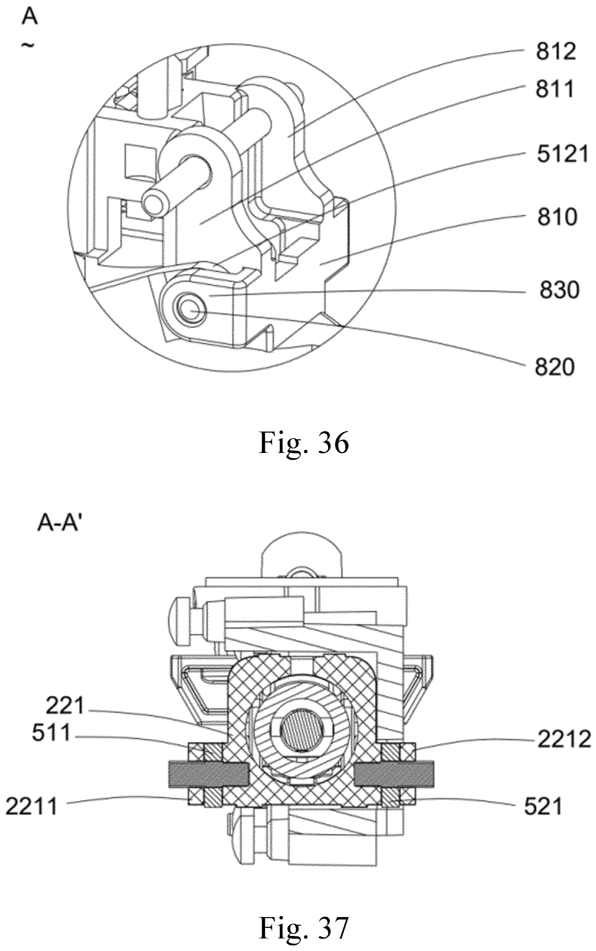

For example, as shown in Figs. 35 and 36, the connecting rod assembly 500 further includes a connecting portion 800, one end of the connecting portion 800 is rotatably connected to the left second connecting rod 512, and the other end of the connecting portion 800 is rotatably connected to the right second connecting rod 522. The connecting portion 800 connects the left second connecting rod 512 to the right second connecting rod 522, so that the left connecting rod 510 and the right connecting rod 520 can move synchronously. The connecting portion 800 is connected to the left second connecting rod 512 and the right second connecting rod 522, respectively; the connecting portion 800 can penetrate the frame 410 or be arranged around the housing of the frame 410. For example, the connecting portion 800 is provided at one end of the frame 410 and is arranged around the housing of the frame 410, and the connecting portion 800 is arranged in a concave shape as a whole and includes a clamp plate 810, a pin shaft 820, a left hinge portion 830, and a right hinge portion. The right hinge portion is disposed corresponding to the left hinge portion 830. For example, the right hinge portion is symmetrically disposed with the left hinge portion 830. The structure of the right hinge portion can refer to that of the left hinge portion 830 shown in Fig. 35. For example, the clamp plate 810 is arranged in a concave shape and includes three plate bodies, namely a left plate 811, a right plate 812 and a connecting plate 813. The left plate 811 is fixed on the left side of the frame 410, the right plate 812 is fixed on the right side of the frame 410, the connecting plate 813 is attached to the frame 410, the left plate 811 and the right plate 812 are respectively arranged on both sides of the connecting plate 813, and the left plate 811 and the right plate 812 are both connected to the frame 410 through fasteners, so that the clamp plate 810 is fixed to the frame 410. For example, the left plate 811, the right plate 812 and the connecting plate 813 are integrally formed, so as to improve the stability of the clamp plate 810. The left hinge portion 830 is provided on the left plate 811, the right hinge portion is provided on the right plate 812, the left hinge portion 830 is rotatably connected to the left second connecting rod 512, and the right hinge portion is rotatably connected to the right second connecting rod 522. For example, the rotatable connection between the left hinge portion 830 and the left second connecting rod 512, and the rotatable connection between the right hinge portion and the right second connecting rod 522 are both realized through the pin shaft 820.

As shown in Figs. 33-36, the left second connecting rod 512 has a left hinge end 5121, the left hinge end 5121 is located between the clamp plate 810 and the left hinge portion 830, specifically, the left hinge end 5121 is located between the left plate 811 and the left hinge portion 830; and a pin shaft 820 penetrates the left hinge portion 830 and the left plate 811, so that the left second connecting rod 512 is rotatably connected to the left hinge portion 830. As shown in Figs. 33-36, the right second connecting rod 522 has a right hinge end 5221, the right hinge end 5221 is located between the clamp plate 810 and the right hinge portion, specifically, the right hinge end 5221 is located between the right plate 812 and the right hinge portion; and a pin shaft 820 penetrates the right hinge end 5221 and the right plate 812, so that the right second connecting rod 522 is rotatably connected to the right hinge portion. In one embodiment, the pin shaft 820 is divided into two portions, one of which rotatably connects the left second connecting rod 512 to the left hinge portion 830, and the other of which rotatably connects the right second connecting rod 522 to the right hinge portion. For example, the pin shaft 820 is a shaft body, and the pin shaft 820 penetrates the left hinge portion 830, the left plate 811, the frame 410, the right plate 812, and the right hinge portion, so that the left second connecting rod 512 is rotatably connected to the left hinge portion 830, and at the same time, the right second connecting rod 522 is rotatably connected to the right hinge portion; and they are integrated with the frame 410, so that the stability of the connection between the left second connecting rod 512 and the right second connecting rod 522 is improved.

As shown in Fig. 35, the left first connecting rod 511 includes a left first hinge hole 5111 and a left middle hinge hole 5112, the left first connecting rod 511 is rotatably connected to the jaw assembly 300 through the left first hinge hole 5111, and the left first connecting rod 511 is rotatably connected to the left second connecting rod 512 through the left middle hinge hole 5112. Correspondingly, the right first connecting rod 521 includes a right first hinge hole and a right middle hinge hole (not shown in the figure), the right first connecting rod 521 is rotatably connected to the jaw assembly 300 through the right first hinge hole, and the right first connecting rod 521 is rotatably connected to the right second connecting rod 522 through the right middle hinge hole. For example, the left first hinge hole 5111 is coaxially arranged with the right first hinge hole, the left middle hinge hole 5112 is coaxially arranged with the right middle hinge hole, and the left hinge end 5121 of the left second connecting rod 512 is coaxially arranged with the right hinge end 5221 of the right second connecting rod 522. Further, for example, the length of the left first connecting rod 511 is the same as the length of the right first connecting rod 521, and the length of the left second connecting rod 512 is the same as the length of the right second connecting rod 522, so that the left connecting rod 510 and the right connecting rod 520 can move in a mirrored way, without affecting the stability of the movement of the connecting rod assembly 500 while improving the stability of the connecting rod assembly 500.

As shown in Figs. 31 and 32, the operating assembly 400 further includes an outer cannula 221, the outer cannula 221 is connected to the jaw assembly 300, the outer cannula 221 is a long rod body, and both the left first connecting rod 511 and the right first connecting rod 521 are rotatably connected to the outer cannula 221; that is, the left first connecting rod 511 is connected to the jaw assembly 300 through the outer cannula 221, and the right first connecting rod 521 is connected to the jaw assembly 300 through the outer cannula 221. When the handle 100 performs an actuation and drives the connecting rod assembly 500, the left first connecting rod 511 and the right first connecting rod 521 advance toward the distal end, and drive the outer cannula 221 to move toward the distal end, so as to cause the jaw assembly 300 to move from the open position to the closed position.

As shown in Figs. 31-34, the outer cannula 221 is arranged in a ring shape, and a left connecting member 2211 and a right connecting member 2212 are provided on left and right sides of the outer cannula 221, respectively; and the left connecting member 2211 and the right connecting member 2212 are oppositely arranged. Being oppositely arranged means that the left connecting member 2211 and the right connecting member 2212 are symmetrically arranged with respect to a reference plane, the axis of the outer cannula 221 is located in the reference plane, and the reference plane is parallel to the left first connecting rod 511 and the right first connecting rod 521. The left first connecting rod 511 is rotatably connected to the left connecting member 2211 through the left first hinge hole 5111, and the right first connecting rod 521 is rotatably connected to the right connecting member 2212 through the right first hinge hole. For example, the left connecting member 2211 is connected to the left first hinge hole 5111 through a pin, and the right connecting member 2212 is connected to the right first hinge hole through another pin.

For example, as shown in Figs. 30 and 38, the handle 100 includes a handle body 120 and a supporting portion 110 connected to the handle 120, the handle body 120 has a gripping portion 121 and a pivot shaft 122. The medical personnel operates the handle 100 by holding the gripping portion 121 of the handle body 120. The handle 100 rotates about the pivot shaft 122 during actuation. The supporting portion 110 is disposed above the pivot shaft 122, and the supporting portion 110 supports the left connecting rod 510 and/or the right connecting rod 520, so that the handle 100 is operably engaged with the left connecting rod 510 and/or the right connecting rod 520.

Referring to Figs. 33 and 30, the handle body 120 is located between the left connecting rod 510 and the right connecting rod 520, the handle body 120 does not interfere with the connecting rod assembly 500 during rotation, the supporting portion 110 is provided on the handle body 120 and extends along the width direction of the handle body 120, the supporting portion 110 is located at the lower side of the connecting rod assembly 500 and abuts against the connecting rod assembly 500, and when the handle 100 performs an actuation, the supporting portion 110 moves to drive the connecting rod assembly 500 to move. In one embodiment, the supporting portion 110 can be disposed on the left side of the handle body 120 to support the left connecting rod 510, and drive the left connecting rod 510 to cause the connecting rod assembly 500 to move. In another embodiment, the supporting portion 110 can be disposed on the right side of the handle body 120 to support the right connecting rod 520. For example, in another embodiment, the supporting portion 110 is disposed on both left and right sides of the handle body 120, the supporting portion 110 supports the left connecting rod 510 and the right connecting rod 520, and simultaneously drives the left connecting rod 510 and the right connecting rod 520 to move, so as to improve the stability of the movement of the connecting rod assembly 500. The supporting portion 110 is a rod body, which penetrates the handle body 120 and protrudes from both sides of the handle body 120. For example, the supporting portion 110 supports the left second connecting rod 512 and the right second connecting rod 522, so as to drive the left connecting rod 510 and the right connecting rod 520 to move. Of course, in other embodiments, the supporting portion 110 can also support the left first connecting rod 511 and the right first connecting rod 521, without being specifically limited. As shown in Figs. 34, 30, 38 and 39, when the handle 100 performs an actuation, the gripping portion 121 of the handle 100 rotates counterclockwise, and the supporting portion 110 rotates counterclockwise, so as to push the left second connecting rod 512 and the right second connecting rod 522 to rotate; the left second connecting rod 512 pushes the left first connecting rod 511 towards the distal end, and the left first connecting rod 511 pushes the outer cannula 221 towards the distal end; similarly, the right second connecting rod 522 pushes the right first connecting rod 521 towards the distal end, and the right connecting rod 521 pushes the outer cannula 221 towards the distal end. The left first connecting rod 511 and the right first connecting rod 521 simultaneously push the outer cannula 221, thereby pushing the jaw assembly 300 to be closed. The connecting rod assembly 500 drives the outer cannula 221 from both the left and right sides, improving the stability of the movement of the outer cannula 221. When the handle 100 moves to the pressed position, the left connecting rod 510 and the right connecting rod 520 are located at or substantially at the dead center, so that the connecting rod assembly 500 is self-locked at the second position.

In the surgical instrument provided by the embodiment of the present disclosure, the movement stability and locking stability of the connecting rod assembly 500 are improved through the arrangement of the left connecting rod 510 and the right connecting rod 520 for the connecting rod assembly 500, so that the closure of the jaw assembly 300 and tissue squeeze are more stable.

For example, as shown in Figs. 38, 39 and 11, the surgical instrument further includes a cutting knife assembly 630, and the operating assembly 400 further includes a cutting knife driving member 600, a mating member 610, and a limiting member 700; the cutting knife driving member 600 is connected to the cutting knife assembly 630, the mating member 610 is connected to the handle 100 and configured to engage with the cutting knife driving member 600. The mating member 610 can drive the cutting knife driving member 600 to move, which in turn drives the cutting knife assembly 630 to advance. When the jaw assembly 300 is at the open position, the tissue can enter into the jaw assembly 300, and when the jaw assembly 300 is at the closed position, the tissue can be squeezed. When the tissue is in the squeezed state, the cutting knife assembly 630 advances to cut the tissue in response to the medical personnel's operation on the operating assembly 400, and the staple cartridge assembly outputs staples to suture the tissue. In response to the first actuation of the handle 100, the connecting rod assembly 500 drives the jaw assembly 300 to switch from the open position to the closed position and holds the jaw assembly 300 at the closed position to squeeze the tissue, and maintains the tissue in the squeezed state. When the handle 100 rebounds from the pressed position to the initial position, the connecting rod assembly 500 is self-locked at the second position and will not move as the handle 100 is released and rebounds, and it always remains in a state in which the jaw assembly 300 is at the closed position. When the handle 100 performs the subsequent actuation, the connecting rod assembly 500 cannot be driven to move and the state in which the jaw assembly 300 is at the closed position cannot be changed. The handle 100 switches from the initial position to the pressed position, and the supporting portion 110 moves with the movement of the handle 100; the supporting portion 110 contacts the connecting rod assembly 500 at the second position only when the handle 100 reaches the pressed position (the endpoint of the movement trajectory of the supporting portion 110), that is, the supporting portion 110 does not contact the connecting rod assembly 500 during movement, and therefore, the handle 100 cannot drive the connecting rod assembly 500 during the subsequent actuation.

As shown in Figs. 38, 39 and 11, the cutting knife driving member 600 is connected to the cutting knife assembly 630, and the mating member 610 is connected to the handle 100. When the mating member 610 engages with the cutting knife driving member 600, the cutting knife driving member 600 can be driven by the handle 100, so as to drive the cutting knife assembly 630.

As shown in Figs. 38, 39 and 11, when the connecting rod assembly 500 is at the first position, the limiting member 700 restrains the mating member 610 so that the mating member 610 disengages from the cutting knife driving member 600; and thus, the first actuation of the handle 100 can only drive the jaw assembly 300 to be closed, but cannot drive the cutting knife assembly 630. When the first actuation is completed and the connecting rod assembly 500 is at the second position, the limiting member 700 releases the restraint on the mating member 610, so as to enable the mating member 610 to engage with the cutting knife driving member 600. The mating member 610 being capable of engaging with the cutting knife driving member 600 means that the mating member 610 moves to a position where the mating member 610 engages with the cutting knife driving member 600. In one embodiment, the mating member 610 automatically move to engage with the cutting knife driving member 600.

In the surgical instrument provided by the embodiment of the present disclosure, only one handle 100 is provided, and the single handle 100 can realize the functions of squeezing and firing; the handle 100 can drive the jaw assembly 300 to be closed for squeezing during the first actuation, and can drive the cutting knife assembly 630 to fire during the subsequent actuation, so that the structure and operation of the surgical instrument are simpler, and the stability of the surgical instrument and the operation convenience of medical personnel are improved.

The limiting member 700 has a locked position and an unlocked position. When the limiting member 700 is at the locked position in Fig. 32, the limiting member 700 restrains the mating member 610 to prevent engagement between the mating member 610 and the cutting knife driving member 600. Before the handle 100 performs the first actuation, the limiting member 700 is at the locked position; in response to the first actuation of the handle 100, the connecting rod assembly 500 simultaneously drives the limiting member 700 to switch from the locked position to the unlocked position in the process that the connecting rod assembly 500 drives the jaw assembly 300 to switch from the open position to the closed position, as shown in Figs. 33, 38 and 39. As shown in Fig. 39, the connecting rod assembly 500 maintains the jaw assembly 300 at the closed position and also maintains the limiting member 700 at the unlocked position. At the unlocked position, the mating member 610 can engage with the cutting knife driving member 600, and during the subsequent actuation, the handle 100 can drive, through the mating member 600, the cutting knife driving member 600 to move, thereby driving the cutting knife assembly 630 to advance for firing.

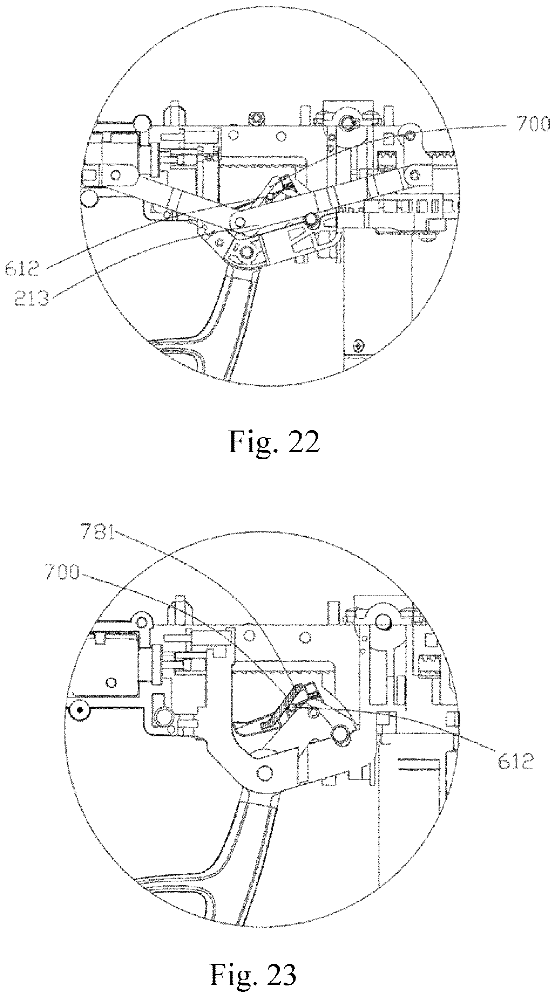

For example, as shown in Fig. 23, when being at the locked position, the limiting member 700 presses against the mating member 610 to exert a force on the mating member 610, so as to restrain the mating member 610, and thus, the mating member 610 is placed at a position where the mating member 610 cannot engage with the cutting knife driving member 600 to prevent engagement between the mating member 610 and the cutting knife driving member 600. When the limiting member 700 is at the unlocked position, the pressing against the mating member 610 is released, so that the mating member 610 can engage with the cutting knife driving member 600.

As shown in Fig. 38, the limiting member 700 is movably arranged on the frame 410 and switches from the locked position to the unlocked position through movement. And the limiting member 700 detaches from the mating member 610 due to movement, so as to release the pressing against the mating member 610, and thus, the mating member 610 can engage with the cutting knife driving member 600. For example, as shown in Figs. 38 and 11, the limiting member 700 is rotatably arranged on the frame 410, and the connecting rod assembly 500 can drive the limiting member 700 to rotate. The limiting member 700 keeps pressing against the mating member 610 during rotating, until the limiting member 700 rotates to the unlocked position and no longer presses against the mating member 610; that is, the limiting member 700 no longer exerts force on the mating member 610, so that the mating member 610 can engage with the cutting knife driving member 600, and can drive the cutting knife assembly 630.

For example, as shown in Fig. 38, the limiting member 700 is substantially elongated as a whole, and includes a body portion 710 and a pressing portion 720; the pressing portion 720 is connected to the body portion 710, and for example, the pressing portion 720 and the body portion 710 are integrated; the body portion 710 is rotatably connected to the frame 410, the pressing portion 720 extends toward the mating member 610, and the limiting member 700 presses against the mating member 610 through the pressing portion 720, so as to prevent engagement between the mating member 610 and the cutting knife driving member 600. Moreover, the limiting member 700 further includes an elastic member (not shown in the figure), and the elastic member can specifically be a torsion spring. The elastic member is arranged between the body portion and the frame to connect the body portion and the frame, and the elastic member exerts elastic force on the limiting member 700 to maintain the limiting member 700 at the locked position and provide a pressing force for the limiting member 700 to press against the mating member 610. When the limiting member 700 is driven by the connecting rod assembly 500 to move to the unlocked position, the elastic member is compressed. At the same time, the elastic member causes the handle 100 to be at the locked position before performing the first actuation. The limiting member 700 further includes a protruding portion 730, and the protruding portion 730 is arranged on the body portion 710. In the process that the connecting rod assembly 500 drives the jaw assembly 300 to switch from the open position to the closed position, the connecting rod assembly 500 acts on the protruding portion 730 and drive the limiting member 700 to rotate through the protruding portion 730, and the connecting rod assembly 500 is not in contact with the body portion 710 or the pressing portion 720 to prevent interference. When the connecting rod assembly 500 maintains the jaw assembly 300 at the closed position, the connecting rod assembly 500 still abuts against the protruding portion 730, so as to maintain the limiting member 700 at the unlocked position.

In one embodiment, as shown in Figs. 38 and 39, the protruding portion 730 is a shaft body and is arranged on the body portion 710, so that the limiting member 700 can always abut against the connecting rod assembly 500 when being pushed to rotate by the connecting rod assembly 500. In another embodiment, as shown in Figs. 4, 5 and 13, the protruding portion 730 is L-shaped and includes two sides, and a corner portion is formed between the two sides; when the protruding portion 730 contacts the connecting rod assembly 500, the corner portion of the L-shaped protruding portion 730 contacts the connecting rod assembly 500 first, and when the limiting member 700 is at the unlocked position, the protruding portion 730 abuts against the connecting rod assembly 500 through one side, so that the contact area with the connecting rod assembly 500 is increased, and further, the stability of being maintained at the unlocked position is improved.

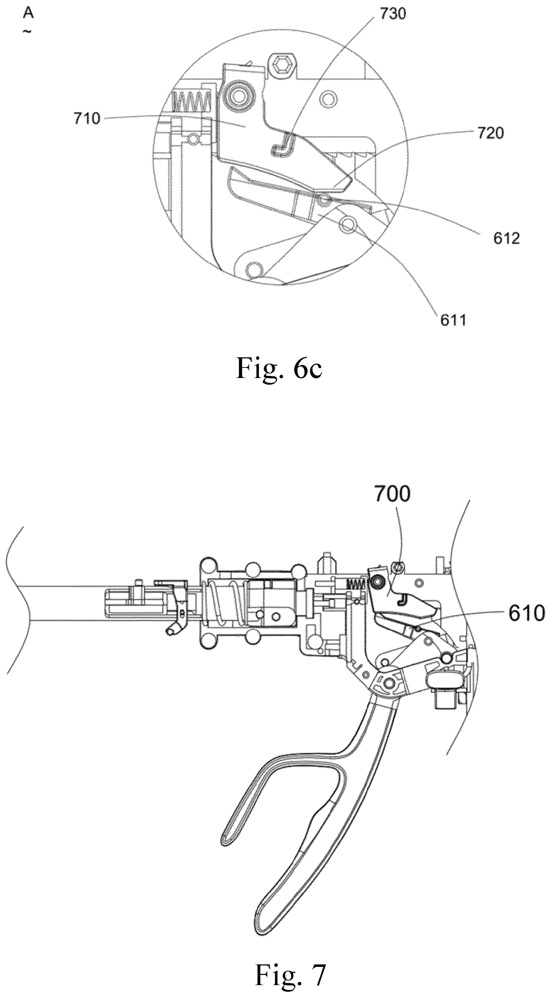

For example, when the limiting member 700 is at the unlocked position, the mating member 610 automatically move to engage with the cutting knife driving member 600, as shown in Figs. 12 and 13; the mating member 610 is movably connected to the handle 100, an elastic member 620 is provided between the handle 100 and the mating member 610, and the mating member 610 moves toward the cutting knife driving member 600 under the action of the elastic force provided by the elastic member 620, that is, the elastic force of the elastic member 620 enables the mating member 610 to move toward the direction of engaging with the cutting knife driving member 600. In the process that the limiting member 700 switches from the locked position to the unlocked position, in conjunction with Figs. 13 and 6c, when the limiting member 700 is at the locked position, the limiting member presses against the mating member 610, the elastic member 620 is compressed, and the mating member 610 cannot engage with the cutting knife driving member 600. When the limiting member 700 is at the unlocked position, the mating member 610 is no longer pressed, and under the action of the elastic force of the elastic member 620, the mating member 610 moves toward the direction of engaging with the cutting knife driving member 600, that is, when the limiting member 700 is switched to the unlocked position, the mating member 610 automatically rebounds under the action of the elastic member 620 to engage with the cutting knife driving member 600.

For example, as shown in Figs. 8, 9 and 12, the mating member 610 includes a pawl 611, and the cutting knife driving member 600 is a toothed member, which can specifically be a rack gear. When the mating member 610 engages with the cutting knife driving member 600, the pawl 611 meshes with the toothed member. The elastic member 620 is a torsion spring, one end of the torsion spring abuts against the handle 100, and the other end of the torsion spring abuts against the pawl 611. The elastic force provided by the torsion spring causes the pawl 611 to rotate to a position of engaging with the toothed member. When the limiting member 700 is at the locked position and presses against the pawl 611, the pawl 611 cannot engage with the toothed member, and the torsion spring is compressed. When the limiting member 700 is switched to the unlocked position, the pressing against the pawl 611 is released, and the pawl 611 rotates under the action of the torsion spring to engage with the toothed member.

When the handle 100 performs the subsequent actuation, it can drive the pawl 611 to move through its own rotation. As shown in Figs. 9 and 10, the handle 100 is rotatably connected to the frame 410 through a hinge shaft, and the pawl 611 is connected to the top end of the handle 100. When being pressed, the handle 100 rotates counterclockwise to cause the pawl 611 to move toward the distal end, thereby driving the toothed member to shift toward the distal end and cause the cutting knife assembly 630 to move toward the distal end for firing. When the handle performs the subsequent actuation, taking the second actuation as an example, when being pressed, the handle 100 drives, through the pawl 611, the toothed member and the cutting knife assembly 630 to move for firing; when being released, the handle 100 rotates clockwise back to its original position, the pawl 611 shifts toward the proximal end, the pawl 611 can only drive the toothed member in a unidirectional manner towards the distal end, the pawl 611 shifts toward the proximal end along the surface of the toothed member, while the toothed member and the cutting knife assembly 630 do not move; that is, when the handle 100 is released, the pawl 611 moves back to its original position, the toothed member and the cutting knife assembly 630 do not move, and the pawl 611 still engages with the toothed member. When performing the third actuation, the movement of the handle 100, pawl 611, the toothed member, and cutting knife assembly 630 is the same as that of the second actuation. It can be seen that in the present embodiment, when the handle 100 performs the subsequent actuation, each actuation will cause the cutting knife assembly 630 to advance a certain distance, and multiple actuations can complete the entire firing of the cutting knife assembly 630.

In addition, it is to be noted that when the limiting member 700 releases the restraint on the pawl 611 and the pawl 611 moves toward the toothed member under the action of the elastic member 620, the pawl 611 has a certain chance of meshing with the toothed member, or has a certain chance of only contacting the toothed member but in a non-meshing state. As shown in Fig. 13, when the pawl 611 is in contact with but does not mesh with the toothed member, in response to the subsequent actuation of the handle 100, the pawl 611 moves toward the distal end, and the moving pawl 611 can mesh with the toothed member and drive the toothed member to move. Therefore, the pawl 611 can stably drive the toothed member to move, thereby driving the cutting knife assembly 630 to move.

For example, as shown in Figs. 13 and 8, the mating member 610 further includes an extension protrusion 612, the extension protrusion 612 extends along the width direction of the pawl 611; the limiting member 700 presses against the mating member 610, specifically, presses against the extension protrusion 612; the setting of the extension protrusion 612 causes the position of the limiting member 700 to be offset from that of the pawl 611, so as to avoid interference between the limiting member 700 and the pawl 611. Before the first actuation of the handle 100, the limiting member 700 is at the locked position, and the pressing portion 720 presses against the extension protrusion 612. When the handle 100 performs the first actuation, the limiting member 700 rotates counterclockwise, the pressing portion 720 rotates synchronously with the limiting member 700, the pawl 611 rotates clockwise to bring the tip of the pawl 611 closer to the toothed member, and the pressing portion 720 always presses against the extension protrusion 612; after the limiting member 700 reaches the unlocked position, the pawl 611 abuts against the toothed member, the pressing portion 720 is located above the extension protrusion 612 and no longer presses against the extension protrusion 612, so that the pawl 611 can drive the toothed member to move.

As can be seen from the above, the first actuation of the handle 100, i.e., the process of being operated from the initial position to the pressed position for the first time, causes the connecting rod assembly 500 to switch from the first position to the second position, and the movement of the connecting rod assembly 500 causes the shift of the outer cannula 221, which in turn causes the jaw assembly 300 to switch from the open position to the closed position; at the same time, the movement of the connecting rod assembly 500 drives the limiting member 700 to switch from the locked position to the unlocked position; however, because of being pressed by the limiting member 700, the mating member 610 cannot engage with the cutting knife driving member 600 and cannot drive the cutting knife assembly 630. That is, the first actuation causes the jaw assembly 300 to be closed, and the cutting knife assembly 630 does not move. After completing the first actuation, the handle 100 is released, the handle 100 rebounds back to the initial position, the connecting rod assembly 500 is locked at the second position, the handle 100 detaches from the connecting rod assembly 500; during the subsequent actuation, the handle 100 is operated from the initial position to the pressed position again, the handle 100 detaches from the connecting rod assembly 500 at the second position, and only at the end of the movement path of the handle 100 will it contact the connecting rod assembly 500, and the connecting rod assembly 500 cannot be driven to move. Therefore, the handle 100 will not change the position of the connecting rod assembly 500 during the subsequent actuation, the connecting rod assembly 500 remains at the second position, and the limiting member 700 remains at the unlocked position. At the same time, during the subsequent actuation, the mating member 610 engages with the cutting knife driving member 600, and the handle 100 can drive the cutting knife driving member 600 to move by driving the mating member 610 to move, thereby driving the cutting knife assembly 630 to fire.

After the surgery, the cutting knife assembly 630 is first retracted to the initial position through a knife retracting mechanism, and then the jaw assembly is opened to release the human tissue. The operating assembly further includes an unlocking assembly (not shown in the figures), the unlocking assembly is configured to unlock the connecting rod assembly 500 at the second position; the unlocking assembly includes an operating portion arranged at the outer side of the housing of the operating assembly 400, and an unlocking portion arranged in the housing of the operating assembly and abutting against the connecting rod assembly 500 at the second position; and the unlocking portion is linked with the operating portion; when medical personnel operates the operating portion, the unlocking portion moves and pushes the connecting rod assembly 500, so that the connecting rod assembly 500 returns back to the first position, and the jaw assembly opens and releases the human tissue. For example, the operating portion can be a button provided on the housing of the operating assembly 400, and the unlocking portion is provided in the operating assembly 400 and is a rod body extending in the up-and-down direction. The button is connected to the rod body. When the button is not operated, the rod body is located above the connecting rod assembly 500, and the connecting rod assembly 500 is self-locked at the second position; when medical personnel operates the button, the rod body moves downward to abut against and push the connecting rod assembly 500 so that the connecting rod assembly 500 is no longer at the second position to release the self-locked state of the connecting rod assembly 500; and the rod body moves further downward to push the connecting rod assembly 500 to switch to the first position, thus opening the jaw assembly 300. For example, when the jaw assembly 300 is opened, the handle 100 is at the initial position, and the connecting rod assembly 500 moves under the action of the rod body, and stops after abutting against the supporting portion 110 of the handle 100; and at this time, the connecting rod assembly 500 is at the first position. When the jaw assembly 300 is in the open state, the connecting rod assembly 500 is at the first position, and the limiting member 700 returns to the locked position under the action of the elastic member.

The surgical instrument provided by the embodiment of the present disclosure is only provided with one handle 100, the handle 100 causes the jaw assembly 300 to be closed and to squeeze during the first actuation, and causes the cutting knife to advance for firing during the subsequent actuation, the entire structure is simple, the stability is high, and the operation is convenient. At the same time, the mating member 610 is always connected to the handle 100, and the mating member 610 can stably drive the cutting knife driving member 600, so that the firing process is more stable. The self-lock of the connecting rod assembly 500 maintains the jaw assembly 300 at the closed position, which does not require operation and does not require the setting of a locking structure, so that the entire structure of the surgical instrument is simple; the connecting rod assembly 500 includes a left connecting rod 510 and a right connecting rod 520, and both the left and right connecting rods move stably and remain at the locked position stably, thereby improving the stability of the closure of the jaw assembly 300 and the stability of tissue squeeze.

In the surgical instrument provided by the embodiment of the present disclosure, the movement stability and locking stability of the connecting rod assembly are improved through the arrangement of the left connecting rod and the right connecting rod for the connecting rod assembly, so that the closure of the jaw assembly and tissue squeeze are more stable.

An embodiment of the present disclosure further provide a surgical instrument which is substantially the same as the surgical instrument in the above embodiments, with the main difference being that the structure of the limiting member 700 is different.

As shown in Figs. 14-20, the limiting member 700 includes a stopper 740. When the limiting member 700 is at the locked position, the stopper 740 is located between the mating member 610 and the cutting knife driving member 600, so as to separate the cutting knife driving member 600 from the mating member 610 to restrain the mating member 610, so that the mating member 610 cannot engage with the cutting knife driving member 600. When the limiting member 700 is at the unlocked position, the stopper 740 detaches from the position between the mating member 610 and the cutting knife driving member 600 to release the restraint on the mating member 610, so as to enable the mating member 610 to engage with the cutting knife driving member 600.

When being at the locked position, the stopper 740 is located between the mating member 610 and the cutting knife driving member 600, and can stably prevent the mating member 610 from engaging with the cutting knife driving member 600. During the first actuation, the mating member 610 does not engage with the cutting knife driving member 600, but still moves under the driving of the handle 100, that is, the mating member 610 slides relative to the cutting knife driving member 600; when the mating member 610 shifts under the driving of the handle, the mating member 610 always abuts against the stopper 740, and the stopper 740 has a sufficient length in the moving direction of the mating member 610, and can stably restrain the mating member 610 and prevent the mating member 610 from engaging with the cutting knife driving member 600.

For example, when the limiting member 700 is at the locked position, the stopper 740 abuts against at least the mating member 610 among the mating member 610 and the cutting knife driving member 600. The stopper 740 abuts against at least the mating member 610 among the mating member 610 and the cutting knife driving member 600, which means that the stopper 740 only abuts against the mating member 610, or the stopper 740 simultaneously abuts against the mating member 610 and the cutting knife driving member 600. In one embodiment, one side (upper side) of the stopper 740 abuts against the cutting knife driving member 600, and the mating member 610 abuts against the other side (lower side) of the stopper 740 under the action of the elastic member 620. In another embodiment, the upper side of the stopper 740 detaches from the cutting knife driving member 600, and the lower side of the stopper 740 abuts against the mating member 610.

For example, as shown in Fig. 14, the limiting member 700 further includes a movement portion 750, the movement portion 750 is movably arranged on the frame 410, and the movement portion 750 is connected to the stopper 740, and can drive the stopper 740 to move, so that the stopper 740 detaches from the position between the mating member 610 and the cutting knife driving member 600. In the process that the closing mechanism 200 drives the jaw assembly 300 to move from the open position to the closed position, the connecting rod assembly 500 drives the movement portion 750 to move, thereby driving the limiting member 700 to switch from the locked position to the unlocked position. Specifically, in the process that the connecting rod assembly 500 switches from the first position to the second position, the connecting rod assembly 500 drives the movement portion 750 to move, and the movement portion 750 drives the stopper 740 to move.

As shown in Figs. 14, 15, 17, 18 and 20, the movement portion 750 is slidably arranged on the frame 410, and includes a sliding portion 751 and a guide portion 752; the sliding portion 751 is slidably connected to the frame 410, the guide portion 752 is connected to the sliding portion 751, the guide portion 752 has a guide inclined surface 761. In the process that the connecting rod assembly 500 switches from the first position to the second position, the connecting rod assembly 500 abuts against and pushes the guide portion 752 to drive the movement portion 750 to move, so as to drive the stopper 740 to detach from the position between the mating member 610 and the cutting knife driving member 600. For example, the frame 410 is provided with a sliding shaft 420 (as shown in Figs. 17 and 20), the sliding portion 751 is arranged in a ring shape and is sleeved around the sliding shaft 420, the sliding portion 751 can move along the axial direction of the sliding shaft 420. The movement portion 750 further includes a spring 770 (as shown in Figs. 17, 18 and 20), the spring 770 is arranged on one side of the sliding portion 751 away from the stopper 740 and is sleeved on the sliding shaft 420 for exerting elastic force on the sliding portion 751. A screw 780 is provided at one end of the sliding shaft 420 away from the frame 410, the screw 780 includes a stud and a nut 782; the stud is screwed into the sliding shaft 420 to engage with the sliding shaft 420, the nut 782 is located on one side of the sliding shaft 420 away from the frame 410, one end of the spring 770 abuts against the nut 782, and the other end of the spring 770 abuts against the sliding portion 751. The guide portion 752 is located below the movement portion 750, the guide inclined surface 761 has a high side and a low side from top to bottom, the high side is located between the low side and the stopper 740 in the front-rear direction; in the process of switching from the first position to the second position, the connecting rod assembly 500 gradually moves upward, firstly abutting against the low side of the guide inclined surface 761, and then pushing the guide portion 752 to move until abutting against the high side of the guide inclined surface 761; in the process of being pushed by the connecting rod assembly 500, the guide portion 752 drives the sliding portion 751 to move in a direction away from the mating member 610 relative to the sliding shaft 420, thereby driving the stopper 740 to move to detach from the position between the mating member 610 and the cutting knife driving member 600. And in this process, the spring 770 is compressed.

For example, the first connecting rod 212 and/or the left second connecting rod 512 in the connecting rod assembly 500 is provided with a mating inclined surface 762. Specifically, the connecting rod contacting the guide portion 752 in the process that the connecting rod assembly 500 switches from the first position to the second position is provided with a mating inclined surface 762 (as shown in Fig. 17). The slope of the mating inclined surface 762 is substantially consistent with the slope of the guide inclined surface 761, so as to better push the guide portion 752 to move.

For example, the stopper 740 is provided in a sheet shape, and the width of the stopper 740 is substantially the same as the width of the cutting knife driving member 600, so as to cover the surface of the cutting knife driving member 600 and prevent the mating member 610 from engaging with the cutting knife driving member 600 while saving the material of the stopper 740 and facilitating the movement of the limiting member 700.

An embodiment of the present disclosure further provides a surgical instrument, which is substantially the same as the surgical instrument in the above embodiments, with the main difference being that the structure of the limiting member 700 is different.

As shown in Figs. 21-24, the limiting member 700 is arranged on one of the left first connecting rod 511, the left second connecting rod 512, the right first connecting rod 521, and the right second connecting rod 522. **In** the process that the connecting rod assembly 500 switches from the first position to the second position, the limiting member 700 moves with the connecting rod assembly 500, and switches from the locked position to the unlocked position, so as to release the restraint on the mating member 610. The limiting member 700 moves with the connecting rod assembly 500 to switch from the locked position to the unlocked position, and the position switching is more stable.

For example, as shown in Figs. 21-24, the left second connecting rod 512 is close to the mating member 610, the limiting member 700 is a rod body provided on the left second connecting rod 512 and forms an angle with the left second connecting rod 512; and the limiting member 700 and the left second connecting rod 512 form an acute angle with each other, so that the limiting member 700 is obliquely provided on the left second connecting rod 512. The limiting member 700 is partially offset from the left second connecting rod 512. The limiting member 700 includes a body portion 770 and a blocking portion 780, the body portion 770 is connected to the left second connecting rod 512, and the projection of the body portion 770 in the vertical direction falls on the left second connecting rod 512; the blocking portion 780 is connected to the body portion 770, for example, can be integrally formed with the body portion 770 and protrude towards the mating member 610. The projection of the mating member 610 in the vertical direction does not fall on the left second connecting rod 512, so as to avoid interference with the moving connecting rod assembly 500.

For example, as shown in Figs. 21-24, the overall shape of the limiting member 700 is obliquely arranged, and the front end and the rear end thereof are both arranged in a flat plate shape. The blocking portion 780 has an abutting surface 781, the abutting surface 781 specifically includes an inclined surface 7811 and two flat surfaces 7812 respectively connected to the front end and the rear end of the inclined surface 7811. When the connecting rod assembly 500 is at the first position, the limiting member 700 is at the locked position, the limiting member 700 abuts against the extension protrusion 612 of the mating member 610 through the abutting surface 781, specifically, abuts against the extension protrusion 612 through the inclined surface 7811; the extension protrusion 612 is arranged in a column shape and can move along the inclined surface 7811; under the action of the elastic member 620, the mating member 610 has a tendency to move toward the cutting knife driving member 600 (toward the front end), and the flat surface 7812 connected to the front end of the inclined surface 7811 can prevent the mating member 610 from detaching from the abutting surface 781.

In the process that the connecting rod assembly 500 moves from the first position to the second position, it drives the limiting member 700 move upward and rotate, so that the extension protrusion 612 of the mating member 610 moves relative to the abutting surface 781. Specifically, the extension protrusion 612 moves toward the rear end along the inclined surface 7811 and detaches from the flat surface 7812 at the rear end. In this process, the elastic member 620 is gradually released. After the connecting rod assembly 500 is at the second position, the limiting member 700 is entirely located above the extension protrusion 612, the elastic member 620 is completely released, the mating member 610 engages with the cutting knife driving member 600 and detaches from the limiting member 700, and the limiting member 700 will not prevent the mating member 610 from subsequently driving the cutting knife driving member 600.

For example, in the embodiment of the present disclosure, the left connecting rod 510 and the right connecting rod 520 in the connecting rod assembly 500 can also be referred to as a first connecting rod assembly and a second connecting rod assembly, respectively; the left first connecting rod 511 and the left second connecting rod 512 in the left connecting rod 510 can be referred to as a first connecting rod of the left connecting rod 510 and a second connecting rod of the left connecting rod 510, respectively, or can also be referred to as a first connecting rod in the first connecting rod assembly and a second connecting rod in the first connecting rod assembly, respectively; the right first connecting rod 521 and the right second connecting rod 522 in the right connecting rod 520 can be referred to as a first connecting rod of the right connecting rod 520 and a second connecting rod of the right connecting rod 520, respectively, and can also be referred to as a first connecting rod in the second connecting rod assembly and a second connecting rod in the second connecting rod assembly, respectively.

For example, in the embodiment of the present disclosure, the left connecting rod 510 and the right connecting rod 520 are relative to the handle 100. The left connecting rod 510 is located on the left side of the handle 100, and the right connecting rod 520 is located on the right side of the handle 100.

It should be understood that although the present specification is described based on embodiments, not every embodiment contains only one independent technical solution. Such a describing way of the present specification is only for the sake of clarity. Those skilled in the art should take the present specification as an entirety. The technical solutions in the respective embodiments may be combined properly to form other embodiments which may be understood by those skilled in the art.

The above are only specific embodiments of the present disclosure, but the protection scope of the present disclosure is not limited thereto. Any person skilled in the art can easily think of changes or substitutions within the technical scope disclosed in the present disclosure. It should be covered within the protection scope of the present disclosure. Therefore, the protection scope of the present disclosure should be subject to the protection scope of the claims.

## Claims

1. A surgical instrument, comprising: an operating assembly, and a jaw assembly and a cutting knife assembly respectively connected to the operating assembly, wherein the operating assembly comprises:
a handle, operable to perform a first actuation and a subsequent actuation;
a closing mechanism, connected to the jaw assembly;
a cutting knife driving member, connected to the cutting knife assembly;
a mating member, connected to the handle; and
a limiting member, having a locked position and an unlocked position, wherein when being at the locked position, the limiting member restrains the mating member to prevent engagement between the mating member and the cutting knife driving member; in response to the first actuation of the handle, the closing mechanism drives the jaw assembly to switch from an open position to a closed position and holds the jaw assembly at the closed position, and drives the limiting member to switch from the locked position to the unlocked position; when the jaw assembly is at the closed position, the limiting member is at the unlocked position; when the limiting member is at the unlocked position, the limiting member releases a restraint on the mating member to enable the mating member to engage with the cutting knife driving member, and in response to the subsequent actuation of the handle, the mating member drives the cutting knife driving member to move, so as to drive the cutting knife assembly.

2. The surgical instrument according to claim 1, wherein when being at the locked position, the limiting member presses against the mating member to restrain the mating member; and when being at the unlocked position, the limiting member detaches from the mating member to release the restraint on the mating member, so as to enable the mating member to engage with the cutting knife driving member.

3. The surgical instrument according to claim 2, wherein the operating assembly further comprises a frame, the limiting member is movably arranged on the frame, and when the limiting member switches from the locked position to the unlocked position, the limiting member moves to detach from the mating member.

4. The surgical instrument according to claim 3, wherein the limiting member comprises a body portion, a pressing portion and a protruding portion, the body portion is rotatably arranged on the frame, the pressing portion is connected to the body portion, and when the limiting member is at the locked position, the pressing portion presses against the mating member; the protruding portion is arranged on the body portion, and the closing mechanism abuts against the protruding portion to drive the protruding portion, so as to further drive the limiting member to switch from the locked position to the unlocked position.

5. The surgical instrument according to claim 1, wherein the limiting member comprises a stopper, and when the limiting member is at the locked position, the stopper is located between the mating member and the cutting knife driving member, so as to restrain the mating member and separate the cutting knife driving member from the mating member; when the limiting member is at the unlocked position, the stopper detaches from a position between the mating member and the cutting knife driving member to release the restraint on the mating member, so as to enable the mating member to engage with the cutting knife driving member.

6. The surgical instrument according to claim 5, wherein when the limiting member is at the locked position, the stopper abuts against at least the mating member among the mating member and the cutting knife driving member.

7. The surgical instrument according to claim 5, wherein the operating assembly further comprises a frame, the limiting member further comprises a movement portion movably arranged on the frame, the movement portion is connected to the stopper, and in a process that the closing mechanism drives the jaw assembly to switch from the open position to the closed position, the closing mechanism drives the movement portion to move, and the movement portion drives the stopper to move to detach from the position between the mating member and the cutting knife driving member.

8. The surgical instrument according to claim 7, wherein the movement portion is slidably arranged on the frame, the movement portion comprises a sliding portion and a guide portion, the sliding portion is slidably connected to the frame, and the guide portion is connected to the sliding portion; in the process that the closing mechanism drives the jaw assembly to switch from the open position to the closed position, the closing mechanism abuts against and pushes the guide portion, so as to drive the movement portion to move.

9. The surgical instrument according to any one of claims 1-8, wherein the closing mechanism comprises a shaft driving member and a shaft assembly, the shaft assembly is connected to the jaw assembly, the shaft driving member is connected to the shaft assembly, when the handle performs the first actuation, the handle is operably engaged with the shaft driving member to drive the shaft driving member to switch from a first position to a second position, so as to further drive the shaft assembly to move and to cause the jaw assembly to switch from the open position to the closed position; and during switching from the first position to the second position, the shaft driving member drives the limiting member to switch from the locked position to the unlocked position.

10. The surgical instrument according to claim 9, wherein the closing mechanism further comprises a locking member, and when the shaft driving member is at the second position, the locking member locks the shaft driving member to hold the jaw assembly at the closed position.

11. The surgical instrument according to claim 10, wherein the locking member comprises a pin, the pin abuts against the shaft driving member when the shaft driving member is at the second position, so as to block a movement of the shaft driving member towards the first position and to lock the shaft driving member.

12. The surgical instrument according to any one of claims 9-11, wherein the shaft driving member comprises a connecting rod assembly, the connecting rod assembly comprises a first connecting rod and a second connecting rod, the first connecting rod is rotatably connected to the shaft assembly, one end of the second connecting rod is rotatably connected to the first connecting rod, and the other end of the second connecting rod is rotatably connected to the frame; the handle is in contact with the first connecting rod or the second connecting rod, such that the handle, during the first actuation, drives the connecting rod assembly to switch from the first position to the second position; when the connecting rod assembly is at the second position, the connecting rod assembly is at or substantially at a dead center, so that the connecting rod assembly is locked at the second position.

13. The surgical instrument according to any one of claims 9-11, wherein the shaft driving member comprises a plurality of connecting rod assemblies, the plurality of connecting rod assemblies comprise a left connecting rod and a right connecting rod, the left connecting rod and the right connecting rod are respectively located on both sides of the frame, the left connecting rod is connected to the right connecting rod, and the handle is operatively engaged with the left connecting rod and/or the right connecting rod to drive the plurality of connecting rod assemblies to move; in response to movement of the plurality of connecting rod assemblies, the jaw assembly moves from the open position to the closed position.

14. The surgical instrument according to claim 13, wherein the left connecting rod comprises a left first connecting rod and a left second connecting rod, the left first connecting rod is connected to the jaw assembly, one end of the left second connecting rod is rotatably connected to the frame, and the other end of the left second connecting rod is rotatably connected to the left first connecting rod; the right connecting rod comprises a right first connecting rod and a right second connecting rod, the right first connecting rod is connected to the jaw assembly, one end of the right second connecting rod is rotatably connected to the frame, and the other end of the right second connecting rod is rotatably connected to the right first connecting rod; and the left second connecting rod is connected to the right second connecting rod.

15. The surgical instrument according to claim 14, wherein the handle drives the plurality of connecting rod assemblies to move from the first position to the second position, when the plurality of connecting rod assemblies are at the first position, the left first connecting rod and the left second connecting rod form a first angle with each other, and the right first connecting rod and the right second connecting rod form the first angle with each other; when the plurality of connecting rod assemblies are at the second position, the left first connecting rod is collinear or substantially collinear with the left second connecting rod, and the right first connecting rod is collinear or substantially collinear with the right second connecting rod.

16. The surgical instrument according to any one of claims 13-15, wherein the limiting member is arranged on the left connecting rod or the right connecting rod, and in a process that the plurality of connecting rod assemblies switch from the first position to the second position, the limiting member moves with the plurality of connecting rod assemblies to switch from the locked position to the unlocked position, so as to release the restraint on the mating member.

17. The surgical instrument according to claim 16, wherein the plurality of connecting rod assemblies further comprise a connecting portion, one end of the connecting portion is rotatably connected to the left second connecting rod, and the other end of the connecting portion is rotatably connected to the right second connecting rod.

18. The surgical instrument according to claim 17, wherein the connecting portion comprises a clamp plate, a left hinge portion and a right hinge portion, the clamp plate is connected to the frame, the left hinge portion and the right hinge portion are respectively arranged at both sides of the clamp plate, the left hinge portion is rotatably connected to the left second connecting rod, and the right hinge portion is rotatably connected to the right second connecting rod.

19. The surgical instrument according to claim 14 or 15, wherein the left first connecting rod comprises a left first hinge hole and a left middle hinge hole, the left first connecting rod is rotatably connected to the jaw assembly through the left first hinge hole, and the left first connecting rod is rotatably connected to the left second connecting rod through the left middle hinge hole; the right first connecting rod comprises a right first hinge hole and a right middle hinge hole, the right first connecting rod is rotatably connected to the jaw assembly through the right first hinge hole, and the right first connecting rod is rotatably connected to the right second connecting rod through the right middle hinge hole;
the left first hinge hole is coaxially arranged with the right first hinge hole, and the left middle hinge hole is coaxially arranged with the right middle hinge hole.

20. The surgical instrument according to any one of claims 14, 15 and 19, wherein the operating assembly further comprises an outer cannula, the outer cannula is connected to the jaw assembly, and both the left first connecting rod and the right first connecting rod are rotatably connected to the outer cannula.

21. The surgical instrument according to any one of claims 1-20, wherein the handle comprises a handle body and a supporting portion connected to the handle body, the supporting portion supports the left connecting rod and/or the right connecting rod, so that the handle is operably engaged with the left connecting rod and/or the right connecting rod.

22. A surgical instrument, comprising: an operating assembly, and a jaw assembly and a cutting knife assembly respectively connected to the operating assembly, wherein the operating assembly comprises:
a frame;
a handle, rotatably connected to the frame, and configured to be operated to perform a first actuation and a subsequent actuation;
a connecting rod assembly, comprising a first connecting rod and a second connecting rod, wherein the first connecting rod is connected to the jaw assembly, one end of the second connecting rod is rotatably connected to the frame, and the other end of the second connecting rod is rotatably connected to the first connecting rod; in response to the first actuation of the handle, the connecting rod assembly switches from a first position to a second position, so as to drive the jaw assembly to be closed; when the connecting rod assembly is at the first position, the first connecting rod and the second connecting rod form an angle with each other, and when the connecting rod assembly is at the second position, the first connecting rod and the second connecting rod are collinear or substantially collinear, so that the connecting rod assembly is self-locked at the second position;
a cutting knife driving member, connected to the cutting knife assembly;
a mating member, connected to the handle; and
a limiting member, wherein when the connecting rod assembly is at the first position, the limiting member restrains the mating member so that the mating member disengages from the cutting knife driving member; when the connecting rod assembly is at the second position, the limiting member releases a restraint on the mating member, so as to enable the mating member to engage with the cutting knife driving member, and in response to the subsequent actuation of the handle, the mating member drives the cutting knife driving member to move, so as to drive the cutting knife assembly.

23. The surgical instrument according to claim 12 or 22, wherein the handle is provided with a supporting portion; the handle supports the first connecting rod or the second connecting rod through the supporting portion to drive the connecting rod assembly to move, so that the connecting rod assembly is switched from the first position to the second position; when the connecting rod assembly is at the second position, the supporting portion detaches from the connecting rod assembly when the handle performs the subsequent actuation.

24. The surgical instrument according to claim 22 or 23, wherein the limiting member is movably arranged on the frame, and the limiting member has a locked position and an unlocked position; when being at the locked position, the limiting member restrains the mating member to prevent engagement between the mating member and the cutting knife driving member; during switching from the first position to the second position, the connecting rod assembly drives the limiting member to move from the locked position to the unlocked position, and when the connecting rod assembly is at the second position, the limiting member is at the unlocked position; when being at the unlocked position, the limiting member releases the restraint on the mating member to enable the mating member to engage with the cutting knife driving member, and in response to the subsequent actuation of the handle, the mating member drives the cutting knife driving member to move, so as to drive the cutting knife assembly.

25. The surgical instrument according to claim 24, wherein when being at the locked position, the limiting member presses against the mating member to restrain the mating member; and when being at the unlocked position, the limiting member detaches from the mating member to release the restraint on the mating member, so as to enable the mating member to engage with the cutting knife driving member.

26. The surgical instrument according to claim 25, wherein the limiting member comprises a body portion, a pressing portion and a protruding portion, the body portion is rotatably arranged on the frame, the pressing portion is connected to the body portion, and when the limiting member is at the locked position, the mating member is pressed by the pressing portion; the protruding portion is arranged on the body portion, and the connecting rod assembly drives the protruding portion, so as to drive the limiting member to switch from the locked position to the unlocked position.

27. The surgical instrument according to claim 22, wherein the limiting member is arranged on the first connecting rod or the second connecting rod, and in a process that the connecting rod assembly switches from the first position to the second position, the limiting member moves with the connecting rod assembly to switch from the locked position to the unlocked position, so as to release the restraint on the mating member.

28. The surgical instrument according to claim 27, wherein the limiting member comprises a body portion and a blocking portion, the body portion is connected to the first connecting rod or the second connecting rod, the blocking portion is connected to the body portion and protrudes toward the mating member, and the blocking portion has an abutting surface; when the limiting member is at the locked position, the abutting surface abuts against the mating member to restrain the mating member; and in a process that the limiting member switches from the locked position to the unlocked position, the abutting surface and the mating member move relatively until the mating member detaches from the abutting surface, so as to release the restraint on the mating member.

29. The surgical instrument according to claim 22, wherein the limiting member comprises a stopper, and when the limiting member is at the locked position, the stopper is located between the mating member and the cutting knife driving member to restrain the mating member, so as to separate the cutting knife driving member from the mating member; when the limiting member is at the unlocked position, the stopper detaches from a position between the mating member and the cutting knife driving member to release the restraint on the mating member, so as to enable the mating member to engage with the cutting knife driving member.

30. The surgical instrument according to claim 29, wherein when the limiting member is at the locked position, the stopper abuts against at least the mating member among the mating member and the cutting knife driving member.

31. The surgical instrument according to claim 29 or 30, wherein the limiting member further comprises a movement portion movably arranged on the frame, the movement portion is connected to the stopper, and in a process that the connecting rod assembly drives the jaw assembly to switch from the open position to the closed position, the connecting rod assembly drives the movement portion to move, and the movement portion drives the stopper to move to detach from the position between the mating member and the cutting knife driving member.

32. The surgical instrument according to claim 31, wherein the movement portion is slidably arranged on the frame, the movement portion comprises a sliding portion and a guide portion, the sliding portion is slidably connected to the frame, and the guide portion is connected to the sliding portion; in the process that the connecting rod assembly drives the jaw assembly to switch from the open position to the closed position, the connecting rod assembly abuts against and pushes the guide portion, so as to drive the movement portion to move.

33. The surgical instrument according to any one of claims 12 and 23-27, wherein the mating member comprises an extension protrusion, and when the connecting rod assembly is at the first position, the limiting member acts on the extension protrusion to restrain the mating member; when the connecting rod assembly is at the second position, the limiting member detaches from the extension protrusion to release the restraint on the mating member.

34. The surgical instrument according to any one of claims 1 and 22-33, wherein the mating member is movably connected to the handle, an elastic member is provided between the handle and the mating member, and the mating member moves toward the cutting knife driving member under an action of an elastic force provided by the elastic member.

35. The surgical instrument according to claim 34, wherein the cutting knife driving member is a toothed member, the mating member comprises a pawl, the pawl is rotatably connected to the handle, the elastic member is a torsion spring, and the pawl moves toward the cutting knife driving member under an action of an elastic force provided by the torsion spring.
